# EUROPEAN PATENT APPLICATION

(11) **EP 2 687 849 A1**
(43) Date of publication of application: **22.01.2014**
(21) Application number: 12177366.7
(22) Date of filing: 20.07.2012
(51) Int. Cl.: G01N 33/574, G01N 33/50

(54) **A process for providing an optimized immune therapy composition**

(71) Applicant: ISA Pharmaceuticals B.V, 3723 MB Bilthoven (NL); Academisch Ziekenhuis Leiden h.o.d.n. LUMC, 2333 ZA Leiden (NL)
(72) Inventor: Krebber, Wilhelmus Johannes Theodorus Alexander, 2333 VN Leiden (NL); Arens, Ramon, 1423 RJ Uithoorn (NL)
(74) Representative: Nederlandsch Octrooibureau

(57) **Abstract**

The present invention relates to a method for measuring the efficacy of an immune therapy composition and to a method for providing an optimized immune therapy composition. Especially by determining in antigen-specific CD8+ T-cells expression (or lack of expressio) of: CD62L, KLRG1, CD154 (CD40L), CD137, CD127, as well as IFNgamma and TNFalpha.

SEQUENCE LISTING

<110>  ISA Pharmaceuticals B.V.
       Academisch Ziekenhuis Leiden h.o.d.n. LUMC

<120>  A process for providing an optimized immune therapy composition

<130>  P6040691EP

<160>  6

<170>  PatentIn version 3.3

<210>  1
<211>  21123
<212>  DNA
<213>  Homo sapiens

<400>  1
cttagctgaa gatgactgac agtgttattt attccatgtt agagttgcct acggcaaccc      60

aagcccagaa tgactatgga ccacagcaaa aatgtgagtt aaccaaggta gcatggaaga     120

cgatgcatag caacagtagg ctatggatga atggggagta gaataagttt aaagcagagg     180

gtgcagaaga attgaggata atttgaggaa atacttttgg ttcagtttaa agtccagagt     240

tggaatatga aaagttggtg tatggtacag gatctgatct taggtctcag gtaaactctt     300

ttctaactgt tcacttcttt gatcgcatct tagaaaccca acttagggca ttgccgttat     360

tccttcttgc tcttagctaa agcgggaact ttgttgggag aattttatct ctttagttat     420

attcggagta accttggacc agctgtttaa attctaggta tggctgtttt tctgactatg     480

gaatgaggag gacaccaata gttttgctgg tggatacgag taagattttt gtcaggaaag     540

aaactgattt acatatctag ttttgttttt tttttttaac ttcacagtgt tgcactagaa     600

tggtgacagg gaagaaaaag tatcaagaaa ctacagataa tagtctctgt gactgaggat     660

ttgaaggcaa aagagccatt tttacataga gccctgagag aatattatct gagacagaca     720

ggtaatagtg taaatgccaa aattaatcct tgattttgta tcgtaggcag tgaactccca     780

caaggtgtat tctttagggt agttccctaa gctttctctc tttttactac atagttttac     840

tattataatt tttaatgtct tttgagagtg ttgccttaat ttgctaaatc attctagtat     900

acttgggata tttagaccta tttctattac cataaattat atagcattgg atatctttat     960

ttgtgtaacc ttttcctatt tttgaattgt atctatgtta atttctaagg aatagaaata    1020

ttacgttaga agtaatgcat atctcaaagt atatgttcaa ggtcacacac acaactggtg    1080

ataaattgga acctagttgg aactatttag aggctttcaa tatatactta aagtaaattt    1140

taaaaattta ctttacttct tgaattggct tgtggttaca caaattcaat tatggtttga    1200

taccttttta aaatttagca taacataaaa atgaacattt gtgtatttca ttaaatgttc    1260

tcctgtgatg caaatgtatt tttaaaagtt tttctagaaa tcttaatgct tttggattca    1320

tcagtccaaa tatttattat gcagaattct ctatttatga aggcatctct cattatgaat    1380

ttttttcttt aagaagttca gaatactcca tagatacaca catatataga ataatttatc 1440
taaaatttaa tcagaaaatt gaagcccaaa taggcaagtt taatttccta attaaatgtt 1500
attaagcaaa ggaatcaaaa ttagaagtta aatatcatct tacttcacag agttttgctc 1560
gttacacgta atttctacac tattgcacta tgagatacct ttgattttaa aataaagatt 1620
tgctgaaaat acttccattt ttcaaattta aaatttagat ttcggttgta tgcaacacag 1680
ctaacatgaa gtagtgtcct attcttaaac ttggcatcaa cttaaactta ctgctctttt 1740
aataacgaca gtttacaaag cacttctgaa atgtagatta tttttattta aattttatgt 1800
atttcaattt cgagtagata atatgtttac atggttcaaa attcaaaagg tacaaaagaa 1860
aatagggtaa aaagtctttt tcctattttg tctccagtga ctgttttttc cattccagag 1920
gcaaacaatg ttatcagatt cttgagtgtg cttgcagaga tatttgtata tcctgttctg 1980
cagaatgttt ctttgtcttg actatatatc ttacaaaata ttccatatca gttaaaaaaa 2040
aagccagttt ttattagaag ctatatattc actttatgga tatatcaaaa atgtatttga 2100
ctggcctttt actgatagaa tgtagattgt attcagtctt ctcctcctac aaatattgct 2160
gccacgaata aatatgacat tttgcacccg cgagtatctg taggaaaaat ttcttaagga 2220
gaaatttcta ggtcaaaggg tacatgcctt tgccgttttg gtagatattg cccaattgtc 2280
ctccaaagag gtaccaattt gtattctcac cacctaagta tgagacagtg taagtcacag 2340
tttcaaagga aaaaagcatt ctttatcttg ataaatatag taagttcttt gatacaggtg 2400
ttggtggtta ccttagactt tagagcaagt tcctgacaca tctagaaaag gaatctttat 2460
ggcctggatc tcagttccca ctaggccaaa ttgctttaag atgcgatatc cttccctgac 2520
tttctctttt ctttcaacct gtcatctgac tcaggattgt gctttgactc tgttgttgca 2580
gcttcctctt ccaggccttc ttgttcttgc cttgtggcaa tagctttggg gcttctgact 2640
gcagttcttc tgagtgtgct gctataccag tggatcctgt gccagggtaa gtgcaaactt 2700
aaaccaaaat taatctttca ttttatatta taaaagcaat ataacatagc agaagctttg 2760
aaaaatcaaa aaataattca aacaaaataa ctctccatct gtatacagct atttcctggg 2820
tatagccttt gaccatgtgc acttgtgtaa gcttgtattt tctttttttct tttgtttttt 2880
tgagacaggg tctccctcaa tcagccaggc tgtaggtgat agatttgtgt gctgctatac 2940
cagcggatcc tgtgccactg tacagtcaca gctcactgca gccttgacct ccttaggctc 3000
aagtgatcca cctcagcctc tcgagtagct gggaccacag gcacatacca ccatgcctgg 3060
ctaatttttg tattattatt attatttttt tttttttgta aagacatttc accatgttgc 3120
ccaggctgat ctcaaactcc tgggctcaag ccatttgccc ttcttggcct cccaaagtgc 3180
tgggattata ggtgtgagcc actgcacctg accatatttt cttaatagaa ctgcagtcaa 3240
gtcctcttaa aatttctccc acctttcttt cttttaatgc aaggctgaga atagttctta 3300
tcgtagtatg ctcataattt tgtatattgc tttttaattt tatgttgcca gaaacatttc 3360

cttactgcca tatttattgg gatgattgaa ttataatatt aatgatgttt aacttttcac 3420
ataatagata tatcataatg taatgaaaca ttctgaattt tttttttttt tttttttgcc 3480
attcaggtgc tttcacattt tctaaaacta atttttaagt ttagttcttc taggacctta 3540
aatctggtga tctagaggac tctcagtttg ttgttttaca gtctagagtc ctagcttagg 3600
tttatatttt taggggatga tagtgaccga ttacacagaa gtgatttatt agggttcttt 3660
tttaaatttt ttttaaaatt ttgagacaga atctcgcaat gttgtttggg ctggagtgca 3720
atggcgtgat ctcggctcac tgcaacctct gcctcccagg ttcaagcgat tctcatgcct 3780
cagcctcccg agtagctggg attacaggca cctgccacca cgcccagcta attttttgta 3840
tttttattag agacggggtt tcactatgtt ggccaggctg gtctcgaact cctgaccttg 3900
tgatctgccc gccttggcct ccccaagtgc tgggattaca ggtgtgagcc attgcgctct 3960
gccgatttat taggattcta agagttcagc tccagggctt ctacctcaga gccaagtccg 4020
acctttgaca aaaaggaact cctagggaag ttgggaattt ctaatccagg ctgagtaatg 4080
tcacagttgt atggaaaaag caaaatctat aaggtgccaa tttaacagct tgatctccag 4140
aacttatatt ggttgtatgc cagtacttcc tagagaacaa ttatctaata tttatatgaa 4200
aaatactact ttccccactt tgtaactccc aaatctagag tagatatata ctctattctg 4260
caattatata tctacacatg tgctcatgga gtataatggc tcatacttgc tcactgtaga 4320
attgtgctta tggcgagttt atgcacatat gtttaagtat tgaaactgaa gtagaaaaat 4380
caatagaatt ttggaatctt ggttggaatt accttgaaag catattcagt tccctttcat 4440
ctttgattgt ttgtttttga gacagtgtct tgctctgtcg cccaggcagg agtgcagtgg 4500
tgtgatcttg gctcactgca acctctgcct cctgggttca agtgattctc atgcctcagc 4560
tcctaagtag ctgggattac aggcgggtgc caccatgccc agctgatttt tttgtatttt 4620
tagtagagat gaggtttcac catgttggct agactgatct tgaactcctg gcctcatgtg 4680
agccacctgc ctcggcctcc caaagtgctg ggattacaag agtgagccac catgcctggt 4740
cagttccctt tcatcttgac agaactatga accaaccatt ttaaaggaat gtggatcttc 4800
cgtatttaaa agagctcggt tagggcattc tatcactttt cactttagca cctgttatgc 4860
ttctctgcca gaaaaccctt ttgaaaaata tcaaagtaag ttctttttgc tgaggtataa 4920
gggttttttt ccagttttca atggtgaggg acagttacat ctgtgataat aaaaaagcaa 4980
aacctttcat tctagttaag tttgcttttc ctaatttctt tttggcattt tcagttgggc 5040
attctgaatg attctgcctg atttaagcaa tatacattat gcaattatgc aatatataca 5100
cagagcaaag acagtacaca gatgcatatc atatgaattc acagttatct ggtgtgtgta 5160
tattacatgt gcacagagat ctctagcaca agtcaagtac ttgctccaga gttttcaggc 5220
agattgttgt atgctttctt ctttgttcct tcagttatag tcatattctt gtgtagtgga 5280

atatatactt agtagatgat ggagttggac attatgagga cattatgagg tattcaaatt 5340
gacaccacgc attgttacac atttctctaa cccttaatct gaaaagtggt ggagcaggga 5400
agacccaggg ctgccatatt ccattttatc tcccatttca tttccaagac tgagtggtcc 5460
ataaagatgt gaaccaggtc ctcttctctc ctaggctcca actactccac ttgtgccagc 5520
tgtcctagct gcccagaccg ctggatgaaa tatggtaacc attgttatta tttctcagtg 5580
gaggaaaagg actggaattc tagtctggaa ttctgcctag ccagagactc acacctcctt 5640
gtgataacgg acaatcagga aatggtaaat gcaaacattt agaaaatgta gggtttttgt 5700
tttgtttttg tttttgtttt taaactgcta tttaaatgta tcatgtatcc tcttttaagg 5760
aacagaagga atctgtgatt tgggggggat actatattct tccccttccc tcttcttctc 5820
tttcctcttc tacttctgcc ttcttccctc tccctcccca ctctttaccc ttatgctgtc 5880
tgacccaaca aacctcagtt acagcaaccc agatgcttgt atagcatgta agtgtgtatg 5940
tggggcaagg aggcagaagg atggtgagtg ttttggagag gaatgagacc tgaaatacag 6000
gtctagttaa aagaagtctt aggtccggaa acctacaggt ccaacaacaa ggcagcagat 6060
tctttttttt tttttttgga cagagtctcc ctctgtcatc caggctggag cacagtggtg 6120
tgatcttggc tcactccaac ctccgcctcc caggttcaag cgcttctcct gcctcagcct 6180
cccaagtagc tggaactaca ggcgtgtgcc accatgcctg gctaattttt tgtattttta 6240
gtagagatgg gatttcacca tgttagccag gatggccttg atctcctgac cttgtgatct 6300
gcccgcctcg gcctcccaaa gtgctgggat tacaggcatg agccaccgtg ccagtcctta 6360
tatagtggaa cttgggaggg agggaaagat agagatcacg gggacagagt gattgtggca 6420
agggcttaaa tgccttcttt taaaaagttt tcccttaatt cactggaatc caataagttc 6480
cagactgcaa tgttgagaaa gttgggatat aaggaagaaa acacgcgtgc acgtgcatgt 6540
gtgtgtgtgt gtggtggcct ccacttcctt caggcccctc agatccttcc tatccaccca 6600
gcaatctctt ggaaaagagc ttgctattgg tcctagacca tctcaaggca atacaaggaa 6660
ctgacatcct gaggttgcct agtataaaaa tcaatcaaat tctaatttgt caacataaat 6720
aacatctata aggaaaacaa gaaatcttag ttttgtgtta gtgctaattt gatactgatt 6780
tttgtgtttt gctattgttt tctgttttct tttattgggt aggatctagt cacagagaac 6840
aatttattga gaattaaact atattaactt accttgggaa aaatccagag gttactctaa 6900
agcagtgggg gagaacatat agttcctaaa ttttatagct acctatataa ctacctatat 6960
aaagcataca gattcttgtt ttctgccaaa ggaacactta ctcctttgga attccacata 7020
gaatctaaac tctaaatcat tccacatttg tgactaactt ttttttttaa agccttctct 7080
aagtgaggtg gcagaatgca ttgagttgta ttaattaaac atgagttctt agttttatgg 7140
cctccagcaa tatagccttg ctttctggac cttgttgtag caactcctat tctcattctg 7200
atatagtgac tctcaggatt tgagctgact aaagtcagta gcagaagtga gccatgaact 7260

tgaaacattg agggcaaaac tgataaacag gagtcgtgag tcatagcaca agacactgcc 7320
acagaaaatt gtggtgtagc agctgctata aatttcacag tgtgatcatg agaggtgagg 7380
agggaaagga tgaagggcac agaatatctg ggaaaatggc aatatttctg tttctctgag 7440
gaacccagag gaagggcagg atcattagag ggattttgtc cttcctctgc ttcctgacat 7500
gcccccattc cttagactat tgttgagtaa aaccagcaga gttacattca ctgcatttcc 7560
aaattgagtt tattttctcc taaggctcct tggggccaag tcagtgctgg agaggcggaa 7620
atgtccctgc tagatgtcat aagttttact tttaacattc ctgtggaatc ttacttgatt 7680
ctctggttta attctttttc ttgctccggc ttttgtcaga ttctgtagaa ccaaattgat 7740
tttacgtgcc ctgctgcgtc tgttgttcta gtgttattat tcttttgttc tcagggcaag 7800
atttacatac tattttgagg tatgtacaat gtttctagta tcatactggg gtctgagcca 7860
ctaccaaaaa agcaagaagt aaaaataaaa aagagagctc tatggatcta ttaaaactga 7920
agtttttcaa actgacattc cattggtgga gagtgctgct agagaatttt gttgctatat 7980
gtagaatcta caggcctatg gaaaatatcc ctcttatgct gttaaacggt catctttcag 8040
tgagatgctt acgtagagtt cacctcctat cttagatcac ttacggcaaa gtgtacaata 8100
ataacagagg ctaccatgga ttgagcactt gctttttgcc agatgtttta tgtatagtat 8160
tctttttttt tttctttttg agatggagtt ttgctcttgt tgcccaggct gaagtgcagt 8220
ggcacaatct cggctcactg caacctctgc ctccagggtt ccagcaattc tcctgcctta 8280
gcctcccaag taactggggt tacaggcgcc tgcctccacg cccgaccttt atgtatagta 8340
ttcttaatcg tcacagcaat tctaaaaagt agagttcaac atccccattt tgttggtgaa 8400
tgaataaaat tcagaggaat tggctgggca tagtggctcc tgcctgtaat cccagcactt 8460
ttgagaggct gaggtgggtg gatcacttga ggtcaggcgt tcgagaccag cctggctaac 8520
atggtgaaac cccatctctg ctaaaaatac aaaaattagc taggcgtgct gttatgcacc 8580
tgtaatccca gcttcttggg aggctaaggc acctgaatcg tttgaaccca ggaggtggag 8640
gctgcagtga gctgagactg caccactgca ctctagctta ggcagcagag tgagacactg 8700
tctcaaaaaa aaaaattcgg agaattaatg agtgtaagtc taaggctaca aatctggcag 8760
agccaaattt taaattctac tccagtgggc atggtggctc acacctgtaa tcctagcact 8820
ttgcgaggcc atggcaggtg gattgcttga acccagaatt caagaccagc ctgggcaaca 8880
tggcaagacc ctgcctctac aaaaaaatac agaaattagc tgggtgtggt ggcgcacacc 8940
tgtggtccca gctactcggg aggctgaggt gagagaatcc cctgaaccca ggaagtagag 9000
gctgcagtta gccgtgatca caccactgca ctccagcctg ggcaacagag cgagactctg 9060
tctcaaaaaa aaaaaaaaaa atccacatca actcaccttc aaagtcactt atagaaatat 9120
cgtatttgac agcttccaga atccattttg catatgttca acaagacaac tttttctact 9180

aattgtcttc tggtttcttt tctatacata tttaaaaatt tttttgaatt gacatacaga 9240
atgtacattg tatgaccgtg gatatttaaa tttatttatt gtgatgaggt ctcactatgt 9300
tgcccaggct ggtcttgaac tcctgggctc aagtgatcct ctcgccttgg tctcccaaag 9360
tgctgggatt ataggcttaa atatatttaa aatagtgaga tgctttctta ttatctcttt 9420
tttttttcta tctgggactt caagattatt cttgatagag aaaataaaat ggaaattaaa 9480
tttctctgtt attttctcat ttttgtgtta acattatggc atcagccctt gcttcttctt 9540
gccctatgag tatatcttta gatgtttgga tatattcaaa ttcttgattt ttcttgccat 9600
ttttccccca ccaagctcag ttcatcctgg atgttgttag cctggttcct tatagttatt 9660
ggtatgttat tttaaagtat gcattcgtta gtgagttccc agtgaaactg ctttgatttc 9720
tttaaatcta ttctgatgct ccttgcccta taatatctct cttcctcatt atcataattt 9780
ggaattatat gatccaactt tactcttgca acaacctctc attcaacttt acctatcttc 9840
ctgaccttgg gattccccga gcttttgatg tcggcttctc acatatttag gatttacatc 9900
taataatatc caaagtttcc ttctttgggt gcagcaatgc aaacactatg gtaacatggt 9960
cacttttctg tatattttta atcaggtaat gaaaacagct tggtttcttt cagacagtca 10020
tgagtgtgct tttactatat ttcttactag ctatgcgaac ttcaacaact ttcttaacct 10080
ttttgactct cagtttcctc atgtacaaag tgaagataaa actttctaga ctgtccgggc 10140
gtggtggctc acgcctgtaa tcccagcact ttgggaggcc gagatgggtg aatgatttga 10200
ggtcagaagt tcaagacctg gccaacatag tgaaacccca tctatactaa aaatacaaaa 10260
attagctggg tgtggtggca cgtttctgta atcccagcta ctcaggaggc tgaggcagga 10320
gaatcacttg agccccggag gcagaggtct cagtgagccg agatcaggcc actgcactcc 10380
agcctgagag acagagtgat actctgtctc aaaaacacaa aaaactcaaa aaacaatata 10440
aagcccagag ccaaaaataa cttttacttt gctcttctgt gtcccaagac atacaatttc 10500
agtaaaatat gcatcacaga gtaaattatg acttgttgaa ttgagtatat tgtttatgag 10560
gaaatctgag atgacatgtc agaagattca aagaaatggc tggaaacaca gcatagatgg 10620
taggggaatt gctttactct ggaggtcttc acagtctggt aatgtgcatc ttttgtcacg 10680
cttctttata atcagattaa gcagctagtg tcaactgcat aacaagggat gggcatgata 10740
agctaggata tttccactat tttttaactt aaaaaattgt gggaaaatat gcatagcata 10800
aaatgtacta ttttaaccat tttcaagtgt acaattgagt ggcattaagt acattcacat 10860
tgttgtacaa ccatcactac catcatctcc agaacttttt catcatcccc agttgaagct 10920
ctgtacctct taaacaataa ctcccctccc tctagcccct ggtaatcact attctactta 10980
ccatatctat gtatttgcct attctgtcct ttagtgtctc acttattcac ttagaacact 11040
gttttcaagg ttcttccgtg ttgtagcatg tatcaaagtt atggctgaat aacattctat 11100
tatatggata tactacattt tgtttgtcca tttattcatc agtggatacc tgggatgctt 11160

caaccttttg actgttgaga gtaaggctgc tgtgaatttg gatatccatc tattgacaga 11220

cactgtttcc acaagctttt aaaaagtgga gcaaatgatg gtttacggag cagggcttac 11280

tgacttttag cattgtgtac ttacacaaaa ttatagggaa aattcttatt gatattaagc 11340

tctagtaatt tgtaacctgt cagattttcc tttgaaattt cttaacgtta tttgcatgtt 11400

tttagatgat ggtaattaat gtgttcatat tataggatat ctgaaaagtc tggaaacagg 11460

ctaaacttat ttttaaacag tatgttggtt acattttcaa gtaatatgct ctatattttt 11520

actcaatctt gagacatgtt ttcaagtgaa acatctctaa atttaaagca atagatataa 11580

ttatttttag accttatgag tactctgtat tgtaaatttt tttcaaatta gtaattacat 11640

cctttgcttt aaatacttca cctgaaaaag tgtctgaaga aaaacatatt aatgatatta 11700

ttttaaaata tgagactgta gggttcccca gtgcctggct ttctttctgt gtattggctg 11760

aaaatcgcag agggccttga ccgctctgtg acccgcccag ctacaggttt ttcccctgca 11820

ggctctaccc aaaccccagc cttgaacatt tccagtcact gataaaggta tctaggttgc 11880

tgctcaaaac actgaaataa actagcccca gccctaagtc agattcctta aaccctcata 11940

aaatctccat ctctgacccc ctggccgtgg acatatgtag gtcaaatgct ccttttctct 12000

ggctgtccat cgtgagtgtt gctgcagccc tctgtaactt cccctaatag atgctttgca 12060

cagatcacct tgacatttag tgctttattc tatggtatcc caaccaaccc catctcatga 12120

tgatttgggg tcctcccttg tgggaattcc cctgtcgctg cttttgggat gatttcagcc 12180

atgggtttgg tgggacaaaa cagagatata ctgcctaaaa atacatttat cctatgtttt 12240

taggtgtttt ggaacccatt attttgtatt ctgcttttat caaatagcat tatttcattc 12300

ttggtttttg ctaagcatta aattattcca ggtaataaaa agttgcaacc agattttatt 12360

ttatttttta tttagtttta aatattattt ttatacgtct tgcatcattc cactattgct 12420

taaatctttt catattctta gataaaattc tccttgtttt caattgtcat gagctgtgag 12480

catatttata gaaatcattt tgcttgcagt tatttattta cattatattc cctaaaatta 12540

agttggctaa tcaaaagata agattttata gttttgatgt attattagtt tttcttaata 12600

tgggttgaaa taacttacat acattctgta aaccatttta atttggaagt ttaaggggta 12660

tgctttgtgc cttaatgata ttttaatttc aattaaaaat actacataca gaagtgtatg 12720

tggaaggtat aaatgttgaa ctaaaaaagc aaaaaaggaa aagaaaagaa aaagtgccct 12780

tgattttatt tgtttgggtt ttatcttcac tttgctttta gggtttgcat tttttctaaa 12840

atataagcaa acattacaaa ctctaagtga cataacacct aagtgttcta gctcacaccc 12900

tctctgggtg acaagagttc tcagttacca cctttaccta tacttttgtt gttgttgttg 12960

ttgttgttgt ttgagactct cacccaggct ggagtgcaat ggcacaatct catctcactg 13020

caacctccac ctcctaggct caaatgaccc tcctgtctca gcctcctaag tagctgggac 13080

tacaggcatg tgccaccatg cccagctaat tttcatattt tttgtattaa tggagttttg 13140

ccatgttgcc caggctggtc tcgaactcct gggctcaaga gattttcctg ccttggcctc 13200

tcaaagtgct ggaagtacag atgtgagcca ctgcaccaga ccgatttgta ctttcatgct 13260

gcttctatat tctctctctt tctaatacac acacacacac acacacacac acacacacac 13320

attatccatg agtacataga atataaggtt attgtactat ggttttgaag gcctcttcca 13380

agtgggaaga ttttcgagtg aaagtgccat aataaacatt ttcaatatcc agcctcacca 13440

atgagaagtt gtaagaggac tgactgatca agtgatttag aattatatgg gtcttgaggg 13500

tagtaaatga gcacaggatt ggggtttgta cgtgggcatt acctaatcct agctctgcag 13560

attctaactg aaatttctga gcaaatcttt atcaatttct gtgcttcatt agctgccgtt 13620

cctcctcttg aaatgaaaga aaaagaaatg gactgttttt ctcattatca gtggagaaat 13680

ggggaatcac tctttttttc cttttgttaa ttttatttat tttttcaaat atttagctta 13740

caatttttga tttgaatata ctcaaatttt atatactttg aatgcagtaa catttatttt 13800

tgaatctttt agatcatggc tatcattcac accaattttg tgaaaatatt aaaaaaaatc 13860

cattcttttt ttcctaattt aaataattct ttaaacaagt tggttaagaa atgcaaaaat 13920

tttaagctat cttgaaaatt acagtgtcat ttcatctgtc gtatacttca ttcactctcc 13980

ggtctacctt cttaccccca gcatgcacag ttaagtgctc cacagagaga aagtaaaaaa 14040

tacagagttt gtgttcagag ggtttttttt ttctttttct tttttattat tattatactt 14100

taagttttag ggtacatgtt cacaatgtgc aggttagtta catatgtata catgtgccat 14160

gctggcgtgc tgcacccatt aactcgttat ttagcattag gtatatctcc taatgctatc 14220

cctcccccct tcccccaccc cacaactgtc cccagagtgt gatgttcccc ttcctgtgtc 14280

catgtgttct cattgttcaa ttcccatcta tgagtgagaa catgcggtgt ttggtttttt 14340

gtccttgcga tagtttactg agaatgatga tttccaattt catccatgtc cctacaaagg 14400

acatgaactc atcatttttt atggctgcat agtattccat ggtgtatatg tgccacattt 14460

tcttaatcca gtctatcatt gttggacatt tgggttggtt ccaagtcttt gctattgtga 14520

atagtgccac aataaacaca cgtgtgcatg tgtctttata gcagcatgat ttatagtcct 14580

ttgggaatat acccagtaat gggatggcag ggtcaaatgg tatttctagt tctagatccc 14640

tgaggaatgg ccacactgac ttccacaatg gttgaactag tttacagtcc caccaacagt 14700

gtaaaagtgt tcctatttct ccacatcctc tccagcacct gttgtttcct gactttttaa 14760

tgattgccat gtgttcagag agttttaact tcttgcagca taatacctaa ttgtgtgttc 14820

tttatttcta tagatactta ttaaacataa ttttttaaaa aagagatggg atcttgctct 14880

gttacccagg ctggagagca atggcatgat cacagctcac tttagcctca aactcctggg 14940

ctcaagtgat cctcctccct gcctctgcct cccaagtaca tgtcaccaag tacaagcaca 15000

tgccaccatg cctggttggc tacatttttt acttttcgta aagatagagt gttactatgt 15060

tgtccaggct ggtcctgaac tcctggcctc aagtaatcct ctcaccttgg cctcccaaat 15120

tgctggtatt acaggcagga gccactgtgc ctggtctgca tttattgagt ataattttat 15180

atatttttct aggtgctagg gatatggtag tcaagagttg ttgctattat aatgtttaca 15240

ttttatttat tatttttaag gaaaatgata ttttaaattt agttttgtta ttaacatttt 15300

tttaaattgg cacataataa ttatatatat agggtattta tccgaaggaa ataaaatcag 15360

tatgttgaag agatatgttc actctcatat gtattgtagc aatattgaca atagccaaga 15420

tatgaaatca accaaaatgt tcaacaacaa ataaatggat aaatgaaact aacacaagaa 15480

cagaaaacca aacaccgcat gttctcactt ataagtagga gttgaacaat gagaacacat 15540

ggacacaggg aggggaacat cacacaccgg gcctgtcggg gggtgggggg caaggggagg 15600

gatagcacta agagaaatac ctaatgtagg tgactagttg atgggtgcag taatccacca 15660

tggcacgtgt atacctatgt aacaaaactg cacattctgc acatgtgccc cagaacttaa 15720

agtataataa taaaaaatga aataaagaaa atgtgtatgt atacaccata gaatactaat 15780

cagccatgga aaataaagaa atcctgtcat tcatagcaga atggatggaa ctagaggaca 15840

ctgtgttaag tgaaataaac caggaacaga aagttaaaca ctgcatgttc tcgcttatgt 15900

gaaaactaaa aaaaatgttg acctcagaac agacgataca agaagctgag aatggctggg 15960

gatggggagg ttagggagag ttatgttaaa ggatacaaaa ttacagctag ataggcagaa 16020

taagttctaa tgttctatag caatggtctc caagatgttt agcaccaggc accagtttcg 16080

tggaagacaa tttttccatg gaccgttgct gggggctggt ttcaggatga aactgttcca 16140

cctcagatca tcaggcatta gttagatttt cttaaggagc aggcaaccta gatccctcac 16200

atgtgcagtt cacgatagtg tttgtactcc tatgagaatc caatgccacc gctgatctgc 16260

tgatctgaca gggggcagaa ctcaggcggt aatgctggcc cgcctagccg ctcacctcct 16320

gctgtgcagc ccagttccta ataggccaca gaccagcacc ttgcatggcc tgagggttgg 16380

ggacccttgt tctatagcac tgcagggtaa tctatagtta acaaccattt attgtatatt 16440

ttcaaatagc tagaagagag gattttgaat gttcccaaca caaagaaata gtaaatgttt 16500

gaagtgatag gctaattatc ctgctctgat cattacacat tgcatacatg ttacgtgtgc 16560

tttcattttc ttcggaagag acagactaaa taaatgaata tggtgatatg tgctttaaag 16620

aaaatgaaac agtatgatat aatagtaaat gtgaatacta ttttagattg gttagttaag 16680

gaaggcctct ttggggaggg aacatttgag ttgatatcta gacctgattg acagccatgt 16740

agaaatctcg ggtaagatat tccaggcaga gggaaaagct atgcaaagac cttaaagcaa 16800

gaatgacctt ggcatgttaa aggaaaagaa agaaggctca tgtggctgga acctaggaag 16860

tgagtaaatg gttagtaggg gctagattgt gtaggactgt gcgtacatag taaggatttt 16920

agattttatt ctatgtgaaa agtgagacta ctggagaaca ttaatcaggg gcacagagtg 16980

attaaaagtt tagcactggg agagaaaagt tggatgatta tttgaaaagg agacttggat 17040
gaattctttc acccacatga ccttgtcttc tcatctctaa aggtgaggat aataatagtt 17100
gcctaccttc caagattgtc aagaatttca taactggaaa gaatcctaga aaatatctaa 17160
cctaaatcct cataaagaag ttgacgctca gccagattaa accacctgct tatcttcaga 17220
taggaggaca gagctaggag gctccagaat tttgtttctg ctttgttttc ctaaatgcca 17280
tactgtatat tcagtatttc aaatgatact gggaatgaac gcaaagggat tttgcaaacc 17340
ttagagtaca atacaaatgt aagagataat tgttttcagg atactgggct ctgagagaac 17400
aaagttatca taaggaatag attatttggg aagaaaagca ctgataaaca agttgtactg 17460
atttttcaaa tgctttgtct aggagccacc acagtcatgt cctgaatgga aacaggactg 17520
aaacccagtg aagagaaaat agaggttatt ctgaccatga gaaatagcac aaaagactcc 17580
ttttctctcc tgtagtgtct ccactagaag tgcaaagagg ctttttgcct ctctttattt 17640
tattttgttt ttttgagaca gagcctcact ccgtcaccca ggctggagtg cagtgatgcc 17700
atctcggctc actgcaatct ctgcctcctg ggttcaagcg attctcatgc ctcagtctcc 17760
caagtagctg ggattacagg tgcccgccac catgcctaat tttttatttt tatttttgtt 17820
tttagtagag acaggctttt tgtaccacat tcaccaggct ggtctcaaac ttctgacctc 17880
aagtgatcca ctagcctcgg cctccctaag tgctgggatc acaggtgtga gccactgtgc 17940
ctggccctcc cttaacttta gaagagcaac tctttatttg tgctgatgca gggtccaagt 18000
aggcaaagag acagttaaga gcagagccca taaatgttta ctcttcagca accatctttc 18060
aggagtggaa taaaaacaac atcaaaatgg gacatctggt ggtatcataa tcaccatagg 18120
cagttgaaat tgtattgctt tcttagtagt ttctagataa aaaagaatat tttattccaa 18180
aggtatttaa ccaattaagc tgtcattaga aaatattgaa agtgtaattg ttaattacac 18240
caacactaaa tcctacaaca aaattacctt ggattatctt tctaaccttt tattttaatc 18300
tattttaatc tttaacattt tattttattt catttataga ggcaattctc tttggatata 18360
aacttcttca tttgaatgtc agactttgca gatcaaagtc ttttatctct gttctgttgg 18420
gtaacgtggc acacataagt ggtgtagcca atttaccttt accttaaact tgttccctta 18480
agaactgttt ttttcttgcc acatacaaga ttgatgagga tgcggaaaat gagacctcag 18540
tggggaaaaa aacaagaata ttgctatagg cttaatcgta atttatatat ttaaggatga 18600
tgggaaagat gaccacttct gcagcaaatg tttagcttct ttcttttgaa ataatctgaa 18660
tacctatcag gtgctccctt taattaagga gaaaattgca atggaaggta gagatgtaat 18720
attatcttat ttgctacatt ataagaatcc agttattgac agaaaggtca attctaggat 18780
gctgctcctc tctacagcta acacatcttt agaattagat gttaaatgag aaattagtta 18840
tttagagtga ataattgtct tagcttgggc tgctctaaca aaataccata gactggatcg 18900
cttgaacaac agatgtttct ctctctcagt tctgaaggct ggaaaatcca acatcaaggt 18960

tctctggctc ctggtgagga cctacttctg gcttgcacat ggctgccttt ttgttgtatc 19020
ctcacttggc aaagagagtg ctttgatctc ttcctcctct tataaggaca ctaattgcat 19080
catggaggcc ccaccctcat ggcctcatct acacctaatt acttgctaaa ggcttcacca 19140
gcagatttca tcacattagg ggttaggggt tcaacatatt gattttagga cgcattcagt 19200
ccttaagagt ggtgatgatg gaggctgtaa ggaatgctag tttgtctttt aattaattac 19260
ttaacttagt attaggaatc caatgtggaa aggaataatt gtttgactgt gacactaggt 19320
cccttttttg gttttcaacc tctcccttag agcctgctcc aagttttcct cagtgaggcc 19380
ttttgctgga ttggtctgag gaacaattct ggctggaggt gggaagatgg atcacctcta 19440
aacttctcaa ggtaaggggc ttcaaaggaa tgcaaaaaaa gagagagagg aaaaaggaaa 19500
gccaaattat gatacttggg gaatagataa agaagagcag atggagagga gaggaaagaa 19560
tgaaaaggag aggaaatagg gaacaaggaa gggaatgaac aatgggtaag ggcaaaaaaa 19620
aaaaaaaaaa ggataacatt ttaaagagag gctgatgaac aattttaatt cttctgctgt 19680
ttgggggaat taggggcctg aatagggaga cagaatttgc ttcattttta tcccttcatg 19740
cctttcaact ccttttctgt gcatgcggcc ttaagtgatt gacttttctg atttcttaca 19800
ggatttcttc taatagcttt gtgcagacat gcggtgccat caacaaaaat ggtcttcaag 19860
cctcaagctg tgaagttcct ttacactggg tgtgtaagaa ggtcagactt tgatagatga 19920
ccactctgtc ctgaccctca gatctgtcat gtatccctaa aaggagggag ctggccactg 19980
gctgttggga aagccatgag tatatagtta gcaaatactg aactttctca gatatggcat 20040
tagatgcaag acaacctcct agggattgat gcctaactga tggattctct ttgagactat 20100
ttagatatta tgtgagcaat ttaaagacca gatctaagca aattttgaaa tagatgtttg 20160
ttttttgtat ttctcagtat ggaaactaat gctgccactc tcatccccgt cccaaccatc 20220
tctgtcaaaa atataccttt ttcatatgat attctgagct aatctgataa aatctatgcc 20280
aatatatact attgcttgtg tactagagaa gtacattatt gctgtactcc tctgtacatt 20340
actgatccct gatggtatat ttctatccta acagtgtccc tttgcagatc aagctttatt 20400
ctgaagaata aacctagctg gcatgctggt gtgtacctgt agtcctagct atttgggaag 20460
ctgaggtggg agggtcgctt gagcccagga gtttgaggct gcagtgagct atgattgtgc 20520
cactgtactc cagcctggga gatagagcaa gactccatct ctaaaaaaaa aaaaaaatgc 20580
taatgtgaga atataaattg tgggaaatga gtgagggcaa ggtggtactt cctccttctg 20640
agctcttcac acgtaatgca aaaacccggt cttaactgat tttgtttttt ttctgagtat 20700
gcatatatgt ggttgaatga accaatgtgt gattgtatct tttccattat gtgactgttt 20760
gacctgcata ttaatttcaa gatagcagtc aattcgataa ggcattttca tagaggaaag 20820
tttacagaaa cagtttatgt ggttggatca ccaaattatc ttaggtacta aggcctcaaa 20880

aataagaaaa actttattat ttctcctcag tagagtttgg acatacataa ggagagaagg 20940

tacagtgatg aaggagacca taattctgta gtgttgatga tcctggatta taatcttttt 21000

ctctttatct ttcatagttt ttttaaaaac atggactgta tcttatctac cactatatcc 21060

caaataccta agatagtgct tacgttcagt gactattaaa taaataaatg gatgaattaa 21120

aaa 21123

<210> 2
<211> 570
<212> DNA
<213> Homo sapiens

<400> 2
atgactgaca gtgttattta ttccatgtta gagttgccta cggcaaccca agcccagaat 60

gactatggac cacagcaaaa atcttcctct tccaggcctt cttgttcttg ccttgtggca 120

atagctttgg ggcttctgac tgcagttctt ctgagtgtgc tgctatacca gtggatcctg 180

tgccagggct ccaactactc cacttgtgcc agctgtccta gctgcccaga ccgctggatg 240

aaatatggta accattgtta ttatttctca gtggaggaaa aggactggaa ttctagtctg 300

gaattctgcc tagccagaga ctcacacctc cttgtgataa cggacaatca ggaaatgagc 360

ctgctccaag ttttcctcag tgaggccttt tgctggattg gtctgaggaa caattctggc 420

tggaggtggg aagatggatc acctctaaac ttctcaagga tttcttctaa tagctttgtg 480

cagacatgcg gtgccatcaa caaaaatggt cttcaagcct caagctgtga agttccttta 540

cactgggtgt gtaagaaggt cagactttga 570

<210> 3
<211> 189
<212> PRT
<213> Homo sapiens

<400> 3

Met Thr Asp Ser Val Ile Tyr Ser Met Leu Glu Leu Pro Thr Ala Thr
1               5                   10                  15

Gln Ala Gln Asn Asp Tyr Gly Pro Gln Gln Lys Ser Ser Ser Ser Arg
            20                  25                  30

Pro Ser Cys Ser Cys Leu Val Ala Ile Ala Leu Gly Leu Leu Thr Ala
        35                  40                  45

Val Leu Leu Ser Val Leu Leu Tyr Gln Trp Ile Leu Cys Gln Gly Ser
    50                  55                  60

Asn Tyr Ser Thr Cys Ala Ser Cys Pro Ser Cys Pro Asp Arg Trp Met
65                  70                  75                  80

Lys Tyr Gly Asn His Cys Tyr Tyr Phe Ser Val Glu Glu Lys Asp Trp
                85                  90                  95

Asn Ser Ser Leu Glu Phe Cys Leu Ala Arg Asp Ser His Leu Leu Val
            100                 105                 110

Ile Thr Asp Asn Gln Glu Met Ser Leu Leu Gln Val Phe Leu Ser Glu
        115                 120                 125

Ala Phe Cys Trp Ile Gly Leu Arg Asn Asn Ser Gly Trp Arg Trp Glu
    130                 135                 140

Asp Gly Ser Pro Leu Asn Phe Ser Arg Ile Ser Ser Asn Ser Phe Val
145                 150                 155                 160

Gln Thr Cys Gly Ala Ile Asn Lys Asn Gly Leu Gln Ala Ser Ser Cys
                165                 170                 175

Glu Val Pro Leu His Trp Val Cys Lys Lys Val Arg Leu
            180                 185

<210>  4
<211>  195
<212>  PRT
<213>  Homo sapiens

<400>  4

Met Thr Asp Ser Val Ile Tyr Ser Met Leu Glu Leu Pro Thr Ala Thr
1               5                   10                  15

Gln Ala Gln Asn Asp Tyr Gly Pro Gln Gln Lys Ser Ser Ser Ser Arg
            20                  25                  30

Pro Ser Cys Ser Cys Leu Val Ala Ile Ala Leu Gly Leu Leu Thr Ala
        35                  40                  45

Val Leu Leu Ser Val Leu Leu Tyr Gln Trp Ile Leu Cys Gln Gly Ser
    50                  55                  60

Asn Tyr Ser Thr Cys Ala Ser Cys Pro Ser Cys Pro Asp Arg Trp Met
65                  70                  75                  80

Lys Tyr Gly Asn His Cys Tyr Tyr Phe Ser Val Glu Glu Lys Asp Trp
                85                  90                  95

Asn Ser Ser Leu Glu Phe Cys Leu Ala Arg Asp Ser His Leu Leu Val
            100                 105                 110

Ile Thr Asp Asn Gln Glu Met Ser Leu Leu Gln Val Phe Leu Ser Glu
        115                 120                 125

Ala Phe Cys Trp Ile Gly Leu Arg Asn Asn Ser Gly Trp Arg Trp Glu
    130                 135                 140

Asp Gly Ser Pro Leu Asn Phe Ser Arg Ile Ser Ser Asn Ser Phe Val
145                 150                 155                 160

Gln Thr Cys Gly Ala Ile Asn Lys Asn Gly Leu Gln Ala Ser Ser Cys
                165                 170                 175

Glu Val Pro Leu His Trp Val Cys Lys Lys Cys Pro Phe Ala Asp Gln
            180                 185                 190

Ala Leu Phe
        195

<210>  5
<211>  35
<212>  PRT
<213>  Artificial

<220>
<223>  Peptide

<400>  5

Gly Gln Ala Glu Pro Asp Arg Ala His Tyr Asn Ile Val Thr Phe Cys
1               5                   10                  15

Cys Lys Cys Asp Ser Thr Leu Arg Leu Cys Val Gln Ser Thr His Val
            20                  25                  30

Asp Ile Arg
        35

<210>  6
<211>  9
<212>  PRT
<213>  Artificial

<220>
<223>  Peptide

<400>  6

Arg Ala His Tyr Asn Ile Val Thr Phe
1               5

## Description

### FIELD OF THE INVENTION

The present invention relates to the fields of medicine and immunology. In particular, it relates to a method for measuring the efficacy of an immune therapy composition and to a method for providing an optimized immune therapy composition.

### BACKGROUND OF THE INVENTION

Antigen specific, particularly tumor antigen specific, immune therapy-induced T-cells, particularly CD8+ T-cells, play a central role in the control of and protection against intracellular pathogens and malignant T-cells. The differentiation of naive CD8+ T-cells into effector and memory cell populations is accompanied with substantial plasticity regarding phenotype and functional capacity (Kaech et al, 2002; Arens et al, 2010), emphasizing the multi-facetted and important role of this T-cell subset. Such heterogeneity among T-cells might be beneficial in defining correlates of immune protection after vaccination, and thus could expedite better prediction of effective vaccine formulations.

Although certain immunotherapeutic strategies of cancers have clinical efficacy, successful therapeutic vaccines are not yet available for the treatment of most tumors (Melief et al, 2008). Importantly, the better the characterization of therapeutic efficacy that reckons with the heterogeneity of T-cell responses, including more precise charting of function, phenotype and differentiation, can help to define correlates of tumor eradication induced by (therapeutic) vaccination. Thus far such immune correlations of vaccine-induced therapeutic activity against tumors are lacking. Studies in the field of microbial immunity have shown that profiles of antigen-specific T-cell responses can be correlated with disease activity and/or protection (Harari et al, 2006; Darrah et al, 2007; Seder et al, 2008).

Therefore, there is an urgent need for correlating characteristics of immune therapy-induced anti-tumor T-cell responses with the efficacy of an immune therapy composition in order to optimize development and design of therapeutic vaccines against cancer.

### SUMMARY OF THE INVENTION

In an aspect, the present invention provides an *ex vivo* method for measuring efficacy of an immune therapy composition in an individual, comprising:
a. providing a population of cells comprising T-cells from a sample obtained from an individual,
b. optionally isolating a population of antigen specific immune therapy-induced T-cells from the population obtained in step (a) and optionally measuring the frequency of antigen specific immune therapy-induced T-cells in the population of immune therapy-induced T-cells,
c. measuring in the population obtained in step (a) or (b) at least one of:
   i. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+, and
   ii. the frequency of at least one of antigen specific IFNgamma producing T-cells, antigen specific TNFalpha producing T-cells and antigen specific IL2 producing T-cells, preferably of antigen specific IFNgamma producing T-cells and antigen specific TNFalpha producing T-cells, more preferably of antigen specific IFNgamma producing T-cells, antigen specific TNFalpha producing T-cells and antigen specific IL2 producing T-cells, more preferably of antigen specific T-cells; and
d. correlating a parameter measured in (b) and/or (c) with the efficacy of the immune therapy composition by comparing the parameter with a reference parameter.

In a further aspect, the present invention provides a method for the preparation of an immune therapy composition comprising:
a. providing a population of cells comprising T-cells from a sample obtained from an individual that has been treated with a first immune therapy composition,
b. optionally isolating a population of antigen specific immune therapy-induced T-cells from the population obtained in step (a) and optionally measuring the frequency of antigen specific immune therapy-induced T-cells in the population of immune therapy-induced T-cells,
c. measuring in the population obtained in step (a) or (b) at least one of:
   i. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+ , and
   ii. the frequency of at least one of antigen specific IFNgamma producing T-cells, antigen specific TNFalpha producing T-cells and antigen specific IL-2 producing T-cells, preferably of antigen specific IFNgamma producing T-cells and antigen specific TNFalpha producing T-cells, more preferably of antigen specific IFNgamma producing T-cells, antigen specific TNFalpha producing T-cells and antigen specific IL-2 producing T-cells, more preferably of antigen specific polyfunctional T-cells, and
d. correlating a parameter measured in (b) and/or (c) with the efficacy of the first immune therapy composition by comparing the parameter with a reference parameter,
e. adapting the first immune therapy composition to the individuals needs to obtain a second immune therapy composition.

In yet a further aspect, the present invention provides an immune composition obtainable by a method according to the previous aspect.

In yet a further aspect, the present invention provides a method of treatment of an individual with an immune therapy composition comprising:
a. administering an immune therapy composition to an individual,
b. performing a method according to any one of the preceding aspects, and optionally
c. adapting the immune therapy composition to the individuals needs.

In yet a further aspect, the present invention provides an immune therapy composition for treatment, prevention, reduction or delay of a disease or condition in an individual, wherein the immune therapy composition is to be administered to the individual, wherein subsequently a method according to any one of the preceding aspects is performed and wherein subsequently the immune therapy composition is adapted to the individuals needs to obtain a second immune therapy composition.

In yet a further aspect, the present invention provides the use of at least one parameter selected from the group consisting of:
a. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+ in a population comprising immune therapy-induced T-cells,
b. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+ that are CD127- in a population comprising immune therapy-induced T-cells,
c. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+ that are CD62L- in a population comprising immune therapy-induced T-cells,
d. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+ that are CD127- and are CD62L- in a population comprising immune therapy-induced T-cells,
e. the frequency of at least one of antigen specific IFNgamma producing T-cells, antigen specific TNFalpha producing T-cells and antigen specific IL-2 producing T-cells, preferably of antigen specific IFNgamma producing T-cells and antigen specific TNFalpha producing T-cells, more preferably of antigen specific IFNgamma producing T-cells, antigen specific TNFalpha producing T-cells and antigen specific IL-2 producing T-cells, more preferably of antigen specific polyfunctional T-cells in a population comprising immune therapy-induced T-cells, and
f. the frequency of antigen specific immune therapy-induced T-cells in a population comprising immune therapy-induced T-cells,
for measuring *ex vivo* the efficacy of an immune therapy composition in an individual.

### DETAILED DESCRIPTION OF THE INVENTION

Surprisingly, it has now been demonstrated that a specific marker is correlated to the efficacy of an immune therapy composition.

Accordingly, in a first aspect the present invention provides a method for measuring efficacy of an immune therapy composition in an individual, comprising:
a. providing a population of cells comprising T-cells from a sample obtained from an individual,
b. optionally isolating a population of antigen specific immune therapy-induced T-cells from the population obtained in step (a) and optionally measuring the frequency of antigen specific immune therapy-induced T-cells in the population of immune therapy-induced T-cells,
c. measuring in the population obtained in step (a) or (b) at least one of:
   i. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+ and
   ii. the frequency of at least one of antigen specific IFNgamma producing T-cells, antigen specific TNFalpha producing T-cells and antigen specific IL2 producing T-cells, preferably of antigen specific IFNgamma and antigen specific TNFalpha producing T-cells, more preferably of antigen specific IFNgamma producing T-cells, antigen specific TNFalpha producing T-cells and antigen specific IL2 producing T-cells, more preferably of antigen specific polyfunctional T-cells, and
d. correlating a parameter measured in (b) and/or (c) with the efficacy of the immune therapy composition by comparing the parameter with a reference parameter.

Herein, in all embodiments of the present invention, an antigen can be any antigen such as a bacterial, viral, parasite or tumor antigen; preferably an antigen is a tumor antigen, preferably a tumor antigen as defined later herein; accordingly, the term "antigen specific" preferably means "tumor antigen specific". The antigen specific cells in the embodiments of the invention are preferably specific for the antigen in the immune therapy composition.

A method according to the present invention for measuring efficacy of an immune therapy composition in an individual can be performed *in vivo, ex vivo* or *in vitro.*

Preferably, in a method according to the invention, in step (b), a population of antigen specific immune therapy-induced T-cells from the population obtained in step (a) is isolated. More preferably, in a method according to the invention, in step (b), a population of antigen specific immune therapy-induced T-cells from the population obtained in step (a) is isolated and/or the frequency of antigen specific immune therapy-induced T-cells in the population of immune therapy-induced T-cells is measured.

In a method according to the invention, in step (c), the frequency of (killer cell lectin-like receptor G1) KLRG1+ T-cells may be measured in a population of immune therapy-induced T-cells or in a population of antigen specific immune therapy-induced T-cells.

In a method according to the invention, preferably, the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+ and the frequency of at least one of antigen specific IFNgamma producing T-cells, antigen specific TNFalpha producing T-cells and antigen specific IL2 producing T-cells, preferably of antigen specific IFNgamma and antigen specific TNFalpha producing T-cells, more preferably of antigen specific IFNgamma producing T-cells, antigen specific TNFalpha producing T-cells and antigen specific IL2 producing T-cells, more preferably of antigen specific polyfunctional T-cells is measured.

In step (c) of a method according to the present invention for measuring efficacy of an immune therapy composition in an individual, the frequency of KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+, that are (IL-7 receptor α) CD127- may be measured.

In step (c) of a method according to the present invention for measuring efficacy of an immune therapy composition in an individual, the frequency of KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+, that are (lymphocyte homing receptor) CD62L-may be measured.

In step (c) of a method according to the present invention for measuring efficacy of an immune therapy composition in an individual, the frequency of KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+, that are CD62L- and CD127- may be measured.

Preferably, in a method according to the invention, measuring the frequency of antigen specific KLRG1+, CD127- and/or CD62L- and/or CD154+ and/or CD137+ T-cells and/or measuring the frequency of antigen specific IFNgamma, TNFalpha and/or IL2 producing T-cells, includes measuring whether such cells are CD3+, CD4+ and/or CD8+.

In an embodiment, in step (c) the frequency of antigen specific KLRG1+/CD62L-/CD127-/CD137+/CD8+ T-cells is measured.

In an embodiment, in step (c) the frequency of antigen specific KLRG1+/CD62L-/CD 127-/CD 154+/CD4+ T-cells is measured.

In an embodiment, in step (c) the frequency of antigen specific KLRG1+/CD62L-/CD127-/CD137+/CD8+ T-cells is measured and the frequency of antigen specific KLRG1+/CD62L-/CD127-/CD154+/CD4+ T-cells is measured.

In an embodiment, in step (c) the frequency of antigen specific KLRG1+/CD62L-/CD127-/CD137+/CD8+ T-cells is measured and the frequency of antigen specific IL2 producing T-cells, frequency of antigen specific IFNgamma producing T-cells and frequency of antigen specific TNFalpha producing T-cells is measured.

In an embodiment, in step (c) the frequency of antigen specific KLRG1+/CD62L-/CD127-/CD154+/CD4+ T-cells is measured and the frequency of antigen specific IL2 producing T-cells, frequency of antigen specific IFNgamma producing T-cells and frequency of antigen specific TNFalpha producing T-cells is measured.

In an embodiment, in step (c) the frequency of antigen specific KLRG1+/CD62L-/CD127-/CD137+/CD8+ T-cells is measured, the frequency of antigen specific KLRG1+/CD62L-/CD127-/CD154+/CD4+ T-cells is measured and the frequency of antigen specific IL2 producing T-cells, frequency of antigen specific IFNgamma producing T-cells and frequency of antigen specific TNFalpha producing T-cells is measured, preferably as CD4/CD8/ IFNgamma/TNFalpha/IL-2 measured together in a single assay..

KLRG1, CD62L and CD137 are known to the person skilled in the art and are *inter alia* described in a review paper by Obar et al, which is herein incorporated by reference (Obar JJ, L. Lefrançois. 2010. Memory CD8+ T-cell differentiation. Ann N Y Acad Sci. 1183: 251-66). A preferred KLRG1 is the human KLRG1 with the amino acid sequence as set forth in SEQ ID NO: 3 and SEQ ID NO: 4 encoded by SEQ ID NO: 1 and SEQ ID NO: 2; SEQ ID NO: 3 is a splice isoform of KLRG1 of 185 amino acids; SEQ ID NO: 4 is 195 amino acids CD154 and CD137 are known to the person skilled in the art and are *inter alia* described in review papers by Kutscher et al and by Snell et al, respectively, which are herein incorporated by reference (Kutscher, S. 2010. MVA-nef induces HIV-1-specific polyfunctional and proliferative T-cell responses revealed by the combination of short- and long-term immune assays. Gene Therapy 17, 1372-1383; Snell, M. 2011. T-cell intrinsic effects of GITR and 4-1BB during viral infection and cancer immunotherapy. Immunological Reviews Vol. 244: 197-217).

The individual for whom the efficacy of an immune composition is measured, is preferably treated with the immune composition before a method according to the present invention for measuring efficacy of an immune therapy composition in an individual is performed. The individual may have been treated once or multiple times with the immune composition before the sample has been taken.

In a method according to the present invention for measuring efficacy of an immune therapy composition in an individual, the sample wherefrom a population comprising immune therapy-induced T-cells is derived, may be a sample that comprises substantially no tumor cells. Accordingly, the present invention provides a convenient method for measuring the efficacy of an immune therapy composition without having to sample the tumor itself or without a tumor sample being available. This is a huge advantage where e.g. sampling would not be possible due to the clinical situation of the individual or where a tumor is difficult to locate or sample, such as in the case of brain, pancreas, colon and lung tumors.

Substantially no tumor cells is herein defined as preferably less than 10%, 5%, 4%, 3%, 2%, 1%, 0.5 %, 0.4%, 0.3%, 0.2%, 0.1%, 0.05%, 0.01%, 0.001% or most preferably less than 0.001% tumor cells compared to the total amount of cells in the sample.

In a method according to the present invention for measuring efficacy of an immune therapy composition in an individual, the individual for whom the efficacy of an immune composition is measured, may be an individual without any clinical signs of a disease to be treated with the immune therapy composition. Accordingly, the present invention provides a convenient method for measuring the efficacy of a vaccine composition in an individual before the individual develops any clinical signs of a disease to be treated. Preferably, the individual is without any signs of a cancer and/or associated tumor to be treated with the immune therapy composition. Examples of cancer include brain cancer, lung cancer, bone cancer, pancreatic cancer, skin cancer, cancer of the head and neck, cutaneous or intraocular melanoma, uterine cancer, ovarian cancer, rectal cancer, cancer of the anal region, stomach cancer, gastric cancer, colorectal cancer, colon cancer, gynecologic tumors (e. g. uterine sarcomas, carcinoma of the fallopian tubes, carcinoma of the endometrium, carcinoma of the cervix, carcinoma of the vagina or carcinoma of the vulva, HPV derived cancer), cancer of the esophagus, cancer of the small intestine, cancer of the endocrine system (e. g. , cancer of the thyroid, parathyroid or adrenal glands), sarcomas of soft tissues, leukemia, myeloma, multiple myeloma, cancer of the urethra, cancer of the penis, prostate cancer, chronic or acute leukemia, solid tumors of childhood, Hodgkin's disease, lymphocytic lymphomas, non- Hodgkin lymphoma, cancer of the bladder, liver cancer, renal cancer, cancer of the kidney or urethra (e. g., renal cell carcinoma, carcinoma of the renal pelvis), or neoplasms of the central nervous system (e. g., primary CNS lymphoma, spinal axis tumors, brain stemgliomas or pituitary adenomas), glioma or fibrosarcoma.

In a method according to the present invention for measuring efficacy of an immune therapy composition in an individual, a population of immune therapy-induced T-cells may comprise CD4+ T-cells. Preferably, the population comprises at least 10%, 20%, 30%, 40%, 50%, 60%, 70%, 80%, 90%, 95%, or at least 99% CD4+ T-cells of the total amount of cells in the population.

In a method according to the invention for measuring efficacy of an immune therapy composition in an individual, a population comprising immune therapy-induced T-cells may comprise CD8+ T-cells. Preferably, the population comprises at least 10%, 20%, 30%, 40%, 50%, 60%, 70%, 80%, 90%, 95%, or at least 99% CD8+ T-cells of the total amount of cells in the population.

In a method according to the present invention for measuring efficacy of an immune therapy composition in an individual, a population comprising immune therapy-induced T-cells may comprise both CD4+ T-cells and CD8+ T-cells. Preferably, the population comprises at least 10%, 20%, 30%, 40%, 50%, 60%, 70%, 80%, 90%, 95%, or at least 99% of both CD4+ T-cells and CD8+ T-cells of the total amount of cells in the population.

In a method according to the present invention for measuring efficacy of an immune therapy composition in an individual, the immune therapy composition may be a vaccine composition comprising any vaccine component known to the person skilled in the art, such as HPV serotype 16 oncoproteins, HPV serotype 18 oncoproteins, gp100, MART-1, MAGE-1, BAGE, GAGE, HAGE, tyrosinase, CEA (cancer embryonic antigen), p53, PSA (prostate specific antigen), PSMA (prostate specific membrane antigen); PRAME, HER2/neu, MAGE-1, MAGE-2, MAGE-3, NY-ESO-1, MUC-1, SART-1 or SART-3, XAGE-1B, Tyrosinase, TERT (telomerase reverse transcriptase), WT1, Survivin-2B, gp75, MDM2, telomerase, al[rho]h-1 fetoprotein, CA125, CA15-3, CA19-9, G250, HER2, BCR-ABL, Ras, PML-RARα, PR1, SSX-2, HSP70 or a peptide analogue derived from any of the above mentioned viral, non-viral, tumor, bacterial or parasite antigens.

In step (d) of a method according to the invention, a parameter measured in (b) and/or (c) is correlated with the efficacy of the immune therapy composition by comparing the parameter with a reference parameter. Preferably, a parameter measured in (c) is used, or a parameter measured in (b) and (c) are used. The reference parameter is preferably the same parameter(s) that has been measured in step (b) and/or (c) of the present invention, but the reference parameter having been measured from a reference sample. The reference sample may be any relevant reference sample known to the person skilled in the art and is preferably a previous sample from the same individual before any clinical signs of disease were apparent or a sample from the same individual before the individual has been treated with the immune therapy composition. An immune therapy composition is considered to be effective if a statistically relevant difference between a parameter as defined herein and a reference parameter is present and can preferably be measured.

The parameter used for correlation is preferably one selected from the group consisting of the following parameters defined herein and measured in a method according to the present invention:
- frequency of antigen specific KLRG1+ T-cells;
- frequency of antigen specific KLRG1+/CD62L- T-cells;
- frequency of antigen specific KLRG1+/CD127- T-cells;
- frequency of antigen specific KLRG1+/CD62L-/CD127- T-cells;
- frequency of antigen specific KLRG1+/CD8+ T-cells;
- frequency of antigen specific KLRG1+/CD62L-/CD8+ T-cells;
- frequency of antigen specific KLRG1+/CD127-/CD8+ T-cells;
- frequency of antigen specific KLRG1+/CD62L-/CD127-/CD8+ T-cells;
- frequency of antigen specific KLRG1+/CD4+ T-cells;
- frequency of antigen specific KLRG1+/CD62L-/CD4+ T-cells;
- frequency of antigen specific KLRG1+/CD127-/CD4+ T-cells;
- frequency of antigen specific KLRG1+/CD62L-/CD127-/CD4+ T-cells;
- frequency of antigen specific KLRG1+ T-cells and frequency of antigen specific TNFalpha producing T-cells;
- frequency of antigen specific KLRG1+/CD62L- T-cells and frequency of antigen specific TNFalpha producing T-cells;
- frequency of antigen specific KLRG1+/CD127- T-cells and frequency of antigen specific TNFalpha producing T-cells;
- frequency of antigen specific KLRG1+/CD62L-/CD127- T-cells and frequency of antigen specific TNFalpha producing T-cells;
- frequency of antigen specific KLRG1+/CD8+ T-cells and frequency of antigen specific TNFalpha producing T-cells;
- frequency of antigen specific KLRG1+/CD62L-/CD8+ T-cells and frequency of antigen specific TNFalpha producing T-cells;
- frequency of antigen specific KLRG1+/CD127-/CD8+ T-cells and frequency of antigen specific TNFalpha producing T-cells;
- frequency of antigen specific KLRG1+/CD62L-/CD127-/CD8+ T-cells and frequency of antigen specific TNFalpha producing T-cells;
- frequency of antigen specific KLRG1+/CD4+ T-cells and frequency of antigen specific TNFalpha producing T-cells;
- frequency of antigen specific KLRG1+/CD62L-/CD4+ antigen specific T-cells and frequency of antigen specific TNFalpha producing T-cells;
- frequency of antigen specific KLRG1+/CD127-/CD4+ antigen specific T-cells and frequency of antigen specific TNFalpha producing T-cells;
- frequency of antigen specific KLRG1+/CD62L-/CD127-/CD4+ antigen specific T-cells and frequency of antigen specific TNFalpha producing T-cells;
- frequency of antigen specific KLRG1+ T-cells, frequency of antigen specific IFNgamma producing T-cells and frequency of antigen specific TNFalpha producing T-cells;
- frequency of antigen specific KLRG1+/CD62L- T-cells, frequency of antigen specific IFNgamma producing T-cells and frequency of antigen specific TNFalpha producing T-cells;
- frequency of antigen specific KLRG1+/CD127- T-cells, frequency of antigen specific IFNgamma producing T-cells and frequency of antigen specific TNFalpha producing T-cells;
- frequency of antigen specific KLRG1+/CD62L-/CD127- T-cells, frequency of antigen specific IFNgamma producing T-cells and frequency of antigen specific TNFalpha producing T-cells;
- frequency of antigen specific KLRG1+/CD8+ T-cells, frequency of antigen specific IFNgamma producing T-cells and frequency of antigen specific TNFalpha producing T-cells;
- frequency of antigen specific KLRG1+/CD62L-/CD8+ T-cells, frequency of antigen specific IFNgamma producing T-cells and frequency of antigen specific TNFalpha producing T-cells;
- frequency of antigen specific KLRG1+/CD127-/CD8+ T-cells, frequency of antigen specific IFNgamma producing T-cells and frequency of antigen specific TNFalpha producing T-cells;
- frequency of antigen specific KLRG1+/CD62L-/CD127-/CD8+ T-cells, frequency of antigen specific IFNgamma producing T-cells and frequency of antigen specific TNFalpha producing T-cells;
- frequency of antigen specific KLRG1+/CD4+ T-cells, frequency of antigen specific IFNgamma producing T-cells and frequency of antigen specific TNFalpha producing T-cells;
- frequency of antigen specific KLRG1+/CD62L-/CD4+ T-cells, frequency of antigen specific IFNgamma producing T-cells and frequency of antigen specific TNFalpha producing T-cells;
- frequency of antigen specific KLRG1+/CD127-/CD4+ T-cells, frequency of antigen specific IFNgamma producing T-cells and frequency of antigen specific TNFalpha producing T-cells;
- frequency of antigen specific KLRG1+/CD62L-/CD127-/CD4+ T-cells, frequency of antigen specific IFNgamma producing T-cells and frequency of antigen specific TNFalpha producing T-cells;
- frequency of antigen specific KLRG1+ T-cells, frequency of antigen specific IL2 producing T-cells, frequency of antigen specific IFNgamma producing T-cells and frequency of antigen specific TNFalpha producing T-cells;
- frequency of antigen specific KLRG1+/CD62L- T-cells, frequency of antigen specific IL2 producing T-cells, frequency of antigen specific IFNgamma producing T-cells and frequency of antigen specific TNFalpha producing T-cells;
- frequency of antigen specific KLRG1+/CD127- T-cells, frequency of antigen specific IL2 producing T-cells, frequency of antigen specific IFNgamma producing T-cells and frequency of antigen specific TNFalpha producing T-cells;
- frequency of antigen specific KLRG1+/CD62L-/CD127- T-cells, frequency of IL2 producing T-cells, frequency of IFNgamma producing T-cells and frequency of TNFalpha producing T-cells;
- frequency of antigen specific KLRG1+/CD8+ T-cells, frequency of antigen specific IL2 producing T-cells, frequency of antigen specific IFNgamma producing T-cells and frequency of antigen specific TNFalpha producing T-cells;
- frequency of antigen specific KLRG1+/CD62L-/CD8+ T-cells, frequency of IL2 producing T-cells, frequency of antigen specific IFNgamma producing T-cells and frequency of antigen specific TNFalpha producing T-cells;
- frequency of antigen specific KLRG1+/CD127-/CD8+ T-cells, frequency of IL2 producing T-cells, frequency of antigen specific IFNgamma producing T-cells and frequency of antigen specific TNFalpha producing T-cells;
- frequency of antigen specific KLRG1+/CD62L-/CD127-/CD8+ T-cells, frequency of antigen specific IL2 producing T-cells, frequency of antigen specific IFNgamma producing T-cells and frequency of antigen specific TNFalpha producing T-cells;
- frequency of antigen specific KLRG1+/CD4+ T-cells, frequency of antigen specific IL2 producing T-cells, frequency of antigen specific IFNgamma producing T-cells and frequency of antigen specific TNFalpha producing T-cells;
- frequency of antigen specific KLRG1+/CD62L-/CD4+ T-cells, frequency of antigen specific IL2 producing T-cells, frequency of antigen specific IFNgamma producing T-cells and frequency of antigen specific TNFalpha producing T-cells;
- frequency of antigen specific KLRG1+/CD127-/CD4+ T-cells, frequency of antigen specific IL2 producing T-cells, frequency of antigen specific IFNgamma producing T-cells and frequency of antigen specific TNFalpha producing T-cells;
- frequency of antigen specific KLRG1+/CD62L-/CD127-/CD4+ T-cells, - frequency of antigen specific IL2 producing T-cells, frequency of antigen specific IFNgamma producing T-cells and frequency of antigen specific TNFalpha producing T-cells;
- frequency of the antigen specific T-cells her above wherein the frequency of antigen specific KLRG1+ T-cells includes the frequency of antigen specific KLRG1+/CD154+, KLRG1+/CD137+ or KLRG1+/CD154+ and CD137+ T-cells.
- frequency of antigen specific KLRG1+/CD62L- and frequency of antigen specific KLRG1+/CD62L-/IFNgamma T-cells is specifically preferred.

Herein, in all embodiments of the present invention, the frequency of an antigen specific T-cell such as, but not limited to, an antigen specific KLRG1+ T-cell, an antigen specific CD127- T-cell, an antigen specific CD62L- T-cell, an antigen specific CD137+ T-cell, an antigen specific CD154+ T-cell, an antigen specific TNFalpha producing T-cell, an antigen specific IFNgamma producing T-cell and an antigen specific IL2 producing T-cell, is preferably depicted as the percentage cells, preferably T-cells, preferably antigen specific T-cells that is positive or negative for a specific marker such as CD3, CD4, CD8, KLRG1, CD127, CD62L T-cell, CD137 CD154, TNFalpha, IFNgamma and IL2, or combinations thereof as described earlier herein or produces or does not produce a specific cytokine (whichever is appropriate) of the total amount of cells in the population analyzed; the percentage is preferably at least 0.1 %, 0.2%, 0.3%, 0.4%, 0.5%, 0.6%, 0.7%, 0.8%, 0.9%, 1%, 2%, 3%, 4% , 5%, 6%, 7%, 8%, 9% , 10%, 11%, 12%, 13%, 14%, 15%, 16%, 17%, 18%, 19%, 20%, 21%, 22%, 23%, 24%, 25%. 26%, 27%, 28%, 29%, 30%.

Herein in all embodiment of the present invention, a cell is considered to be positive or negative for a specific marker, produces or does not produce a specific cytokine (whichever is appropriate) when a statistically relevant difference between the subject cell and a reference cell is present. The person skilled in the art knows how to measure the markers and cytokines herein. Preferably, a method of measurement as described herein is used.

To distinguish between positivity and negativity of specific markers (markers such as KLRG1, CD127, CD62L T-cell, CD137 CD154, TNFalpha, IFNgamma and IL2) on or in antigen specific T cells , comparisons with iso-type control antibodies (commercially available) are routinely used. Commercial flow cytometry instruments (e.g. LSR II BD Biosciences) are well capable to accomplish the analysis of the described markers. Compensation of the fluorochrome-conjugated antibodies is routinely performed before acquisition of the samples and can be performed manually but also using the auto-compensation software of the manufacturer or Flowjo software. The person skilled in the art how to use flow cytometry. Detailed protocols for flow cytometric analysis are provided by manufacturers of flow cytometry equipment (e.g. BD Biosciences) and have been extensively described in the literature. Herewith we refer only to recent articles describing (multiparameter) flow cytometric analysis (cell surface staining and intracellular cytokine staining), which are hereby incorporated by reference.: Maecker HT., 2009; Lovelace P et al, 2011; Foster B et al, 2007; Maecker HT et al, 2005.

Analysis of the multiparametric flow cytometric data is usually manually performed using stand-alone software packages (such as Flowjo (www.treestar.com) or using software supplied by the flow cytometer manufacturer (e.g. BD Biosciences).

Herein, in all embodiments of the present invention, an individual is preferably a mammal such as a rodent, a non-human primate, a dog, a cat more preferably a human. The individual may be healthy and/or may be at risk of developing a disease or condition or may be an individual with a disease.

Herein, in all embodiments of the present invention, a "population of cells" is defined as at least two cells; preferably, a population of cells comprises at least 1E+01, 1E+02, 1E+03, 1E+04, 1E+05, 1E+06, or at least 1 E+07 cells. Said population may be an isolated population or said population may be present in a composition.

Herein, in all embodiments of the present invention, a "sample" is defined as a specimen obtained from an individual, preferably comprising immune therapy-induced T-cells. The specimen may be from any tissue or body fluid or excretion, such as, but not limited to blood, urine, lymph node, spleen and tumor tissue. When a method according to the invention is performed as an *ex vivo* method, the sample has already been obtained. The sample may be or may have been obtained by any sampling method known by the person skilled in the art.

Herein, in all embodiments of the present invention, a population comprising immune therapy-induced T-cells may be provided for by any means known to the person skilled in the art. The sample itself may be used. Alternatively, the sample maybe enriched for immune therapy-induced T-cells and/or for antigen specific T-cells using means known to the person skilled in the art. For instance, white blood cells may be separated from whole blood by gradient centrifugation and white blood cells may be further enriched for T-cells, CD8+ T-cells and/or CD4+ T-cells. Methods to enrich for such T-cells are known to the person skilled in the art. Herein, in all embodiments of the present invention, a population comprising antigen specific immune therapy-induced T-cells may isolated from the population obtained in step (a) using any means known to the person skilled in the art. A preferred method is FACS sorting using tetramer staining using tetramers for the specific tumor antigen. The frequency of antigen specific immune therapy-induced T-cells in said population can be measured using any means known to the person skilled in the art, but is preferably the same method as used for isolating the antigen specific immune therapy-induced T-cells. By counting the antigen specific immune therapy-induced T-cells and the starting population, the frequency can be measured.

Herein, in all embodiments of the present invention, the frequency of antigen specific KLRG1+ T-cells, CD154+ T-cells, CD137+ T-cells, CD62L- T-cells and CD127- T-cells may be measured using any means known by the person skilled in the art. Preferably, the frequency of KLRG1+ T-cells, CD154+ T-cells, CD137+ T-cells, CD62L- T-cells and CD127- T-cells is measured using FACS analysis, preferably as described in the examples herein.

Herein, in all embodiments of the present invention, the frequency of antigen specific IFNgamma producing T-cells, TNFalpha producing T-cells, IL2 producing T-cells, and/or T-cells producing IL2, IFNgamma and/or TNFalpha simultaneously may be measured using any means known by the person skilled in the art, such as, but not limited to ELISPOT, FACS analysis and intracellular cytokine staining FACS analysis. Preferably, the frequency of IFNgamma producing T-cells, TNFalpha producing T-cells and IL2 producing T-cells is measured using the FACS method of intercellular cytokine staining as described in the examples herein.

Herein, in all embodiment of the present invention, antigen specific T-cells producing at least two of IL2, IFNgamma and TNFalpha simultaneously are referred to as "polyfunctional T-cells"; preferably such cells produce IFNgamma and TNFalpha simultaneously.

In all methods according to the invention, multicolor FACS analysis such as described in the examples herein is a preferred method to analyze multiple parameters simultaneously. For example, CD3, CD4, CD8, KLRG1, CD62L, CD127, CD154, CD137 and/or antigen specific tetramer can be analyzed simultaneously using antibodies labeled with different colors. Cytokines such as, IFNgamma, TNFalpha and IL2 are preferably measured intracellularly according to the examples herein.

Herein, in all embodiments of the invention, the assays to determine the frequency of antigen specific KLRG1+ T-cells, CD154+ T-cells, CD137+ T-cells, CD62L- T-cells and CD127- T-cells, IFNgamma producing T-cells, TNFalpha producing T-cells, IL2 producing T-cells, and/or polyfunctional T-cells may be performed on cells directly taken from the sample; alternatively the T-cells may be re-stimulated first using an immune therapy composition or antigen according to the invention.

Herein, in all embodiments of the present invention, an immune therapy composition is defined as any composition that is able to elicit an immune response in an individual; preferably such immune composition is able to elicit an immune response against a specific antigen which can be any bacterial, viral, parasite or tumor antigen; preferably such antigen is a tumor antigen. A tumor antigen can be any tumor antigen known to the person skilled in the art, such as, but not limited gp100, MART-1, MAGE-1, BAGE, GAGE, HAGE, tyrosinase, CEA (cancer embryonic antigen), p53, PSA (prostate specific antigen), PSMA (prostate specific membrane antigen); PRAME, HER2/neu, MAGE-1, MAGE-2, MAGE-3, NY-ESO-1, MUC-1, SART-1 or SART-3, XAGE-1B, Tyrosinase, TERT (telomerase reverse transcriptase), WT1, Survivin-2B, gp75, MDM2, telomerase, al[rho]h-1 fetoprotein, CA125, CA15-3, CA19-9, G250, HER2, BCR-ABL, Ras, PML-RARα, PR1, SSX-2, HSP70 or a peptide analogue derived from any of the above mentioned viral, non-viral, tumor, bacterial or parasite antigens. Parasite antigens may be derived from protozoa, nematoda, trematoda or cestoda, such as Cryptosporidium hominis or parvum; Schistosoma haematobium, mansoni or japonicum; Plasmodium falciparum, malariae, vivax or ovale; Leishmania major, tropica, aethiopica, mexicana, donovani, infantum or braziliensis; Toxoplasma Gondii. Bacterial antigens may be antigens derived from Mycobacterium Tuberculosis, Streptococcus pneumoniae, Staphylococcus Aureus, Vibrio cholera, Neisseria meningitides, tetanus and Helicobacter Pylori. Antigens derived from infectious agents that cause diseases such as cancers and/or premalignant conditions include HPV, which causes diseases such as genital warts, a cervical cancer, head and neck cancer, Penile cancer, Vulva cancer, Anal cancer, nasopharyngeal cancer, CIN, VIN, PIN, VAIN and AIN, HCV and HBV, which are involved in liver carcinoma, SV40, which is involved in mesothelioma, HTLV-1, which is involved with T cell leukemia/lymphoma, Merkel cell virus, which is involved with Merkel cell carcinoma and KSV, which is involved with Kaposi sarcoma. Viral antigens may be influenza virus antigen, such as for example HA: haemaglutinin or neuraminidase antigen; human papilloma virus (HPV) antigen, such as E2, E6, E7; human immunodeficiency virus (HIV) antigen, such as for example GP120, GP140, GP160; vesicular stomatitis virus antigen, for example vesicular stomatitis virus glycoprotein; cytomegalovirus (CMV) antigen; hepatitis virus antigens, such as for example hepatitis A(HAV), B(HBV), C(HCV), D(HDV) and G(HGV): L-HBsAg, S-HBsAg, M-HBsAg, pre S; respiratory syntytial virus (RSV) antigen; SV40 virus antigen, such as Large T, small T; EBV antigen, such as EBNA, Kaposi Sarcoma Virus (KSV) antigen, Human T-Lymphotropic Virus-1(HTLV-1) antigen, Merkel cell virus (MCV) antigen or herpes simplex virus antigen.

The HPV strains from which the antigen or peptide used derived is preferably a high risk HPV serotype, such as serotypes 16, 18, 31, 33 or 45, more preferably from the serotype 16, 18, 31 or 33, most preferably from serotype 16 or 18, of which 16 is most preferred.

An immune therapy composition may in all embodiments according to the invention be any immune therapy composition available to the person skilled in the art such as, but not limited to, a vaccine composition. A vaccine composition is a preferred immune therapy composition and may be any vaccine composition known to the person skilled in the art and comprises at least a source of an antigen and a pharmaceutical excipient. The source of antigen may be any source of antigen known to the person skilled in the art, such as, but not limited to tumor (of viral or non-viral origin), a bacterium or virus, a parasite or a peptide, a polypeptide or polypeptide subunit, such as the antigens defined earlier herein. A pharmaceutical excipient may be any pharmaceutical excipient known to the person skilled in the art, such as, but not limited to those described in Remington; The Science and Practice of Pharmacy, 21st Edition 2005, University of Sciences in Philadelphia. A vaccine composition may preferably comprise as a pharmaceutical excipient at least one immune response stimulating compound or adjuvant. Advantageously, the vaccine composition may additionally comprise one or more synthetic adjuvants. Such adjuvant may be admixed to the vaccine composition. Particularly preferred are those adjuvants that are known to act via the Toll-like receptors and/or via a RIG-I (Retinoic acid- Inducible Gene-1) protein and/or via an endothelin receptor. Immune modifying compounds that are capable of activation of the innate immune system can be activated particularly well via Toll like receptors (TLR' s), including TLR' s 1 - 10. Compounds capable of activating TLR receptors and modifications and derivatives thereof are well documented in the art. TLR1 may be activated by bacterial lipoproteins and acetylated forms thereof, TLR2 may in addition be activated by Gram positive bacterial glycolipids, LPS, LPA, LTA, fimbriae, outer membrane proteins, heat shock proteins from bacteria or from the host, and Mycobacterial lipoarabinomannans. TLR3 may be activated by dsRNA, in particular of viral origin, or by the chemical compound poly(I:C). TLR4 may be activated by Gram negative LPS, LTA, Heat shock proteins from the host or from bacterial origin, viral coat or envelope proteins, taxol or derivatives thereof, hyaluronan containing oligosaccharides and fibronectins. TLR5 may be activated with bacterial flagellae or flagellin. TLR6 may be activated by mycobacterial lipoproteins and group B Streptococcus heat labile soluble factor (GBS-F) or Staphylococcus modulins. TLR7 may be activated by imidazoquinolines. TLR9 may be activated by unmethylated CpG DNA or chromatin - IgG complexes. In particular TLR3, TLR7 and TLR9 play an important role in mediating an innate immune response against viral infections, and compounds capable of activating these receptors are particularly preferred. Particularly preferred adjuvants comprise, but are not limited to, synthetically produced compounds comprising dsRNA, poly(I:C), unmethylated CpG DNA which trigger TLR3 and TLR9 receptors, IC31, a TLR 9 agonist, IMSAVAC, a TLR 4 agonist, Montanide ISA-51, Montanide ISA 720 (an adjuvant produced by Seppic 7, France) and TLR 2 agonist Pam3Cys, preferably a Pam3cys lipopeptide, or modifications thereof. RIG-I protein is known to be activated by ds-RNA just like TLR3 (Immunity, (2005), 1: 19-28). In another preferred embodiment, the synthetic adjuvant compounds are physically linked to the peptides of the invention. Physical linkage of adjuvants and costimulatory compounds or functional groups to the HLA class I and HLA class II epitope comprising peptides provides an enhanced immune response by simultaneous stimulation of antigen presenting cells, in particular dendritic cells that internalize, metabolize and display antigen. Another preferred immune modifying compound is an inhibitor of an endothelin receptor such as BQ-788 (Buckanovich RJ et al. Nature Medicine (2008), 14:28-36, Ishikawa K, PNAS (1994) 91:4892). BQ-788 is N-cis-2,6-dimethylpiperidinocarbonyl-L-gamma-methylleucyl-D - 1 - methoxycarbonyltryptophanyl-D-norleucine. However any derivative of BQ-788 or modified BQ-788 compound is also encompassed within the scope of this invention. Furthermore, the use of antigen presenting cell (co)stimulatory molecules, as set out in WO99/61065 and in WO03/084999 is also preferred. In particular the use of 4-1 -BB and/or CD40 ligands, agonistic antibodies, OX40 ligands or functional fragments and derivates thereof are preferred. Other preferred adjuvants are CpG (TLR9 ligand) (Klinman et al, 2006), polyinosinic-polycytidylic acid stabilized by Lysine and carboxymethylcellulose (Poly ICLC, TLR3 ligand, Oncovir, Inc., United States) (Caskey etal, 2011), different forms of LPS (TLR4 ligand), e.g. N. meningitidis wild-type L3-LPS and mutant penta-acylated LpxL1-LPS (Netherlands Vaccine Institute, The Netherlands) (Fransen et al, 2007) and Montanide-ISA51 (Seppic France) (Chiang et al, 2011). Various adjuvants are reviewed in Coffman et al, 2010.

A method according to the present invention for measuring efficacy of an immune therapy composition in an individual can conveniently be used to optimize development and design of therapeutic vaccines against cancer.

Accordingly, in a second aspect, the present invention provides a method for the preparation of an immune therapy composition comprising:
a. providing a population of immune therapy-induced T-cells from a sample obtained from an individual that has been treated with a first immune therapy composition,
b. optionally isolating a population of antigen specific immune therapy-induced T-cells from the population obtained in step (a) and/or optionally measuring the frequency of antigen specific immune therapy-induced T-cells in the population of immune therapy-induced T-cells,
c. measuring in the population obtained in step (a) or (b) at least one of:
   i. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+, and
   ii. the frequency of at least one of antigen specific IFNgamma producing T-cells, antigen specific TNFalpha producing T-cells and antigen specific IL-2 producing T-cells, preferably of antigen specific IFNgamma producing T-cells and antigen specific TNFalpha producing T-cells, more preferably of antigen specific IFNgamma producing T-cells, antigen specific TNFalpha producing T-cells and antigen specific IL2 producing T-cells, more preferably of antigen specific polyfunctional T-cells, and
d. correlating a parameter measured in (b) and/or (c) with the efficacy of the first immune therapy composition by comparing the parameter with a reference parameter,
e. adapting the first immune therapy composition to the individuals needs to obtain a second immune therapy composition.

The individual may have been treated once or multiple times with the immune composition before the sample has been taken.

Steps (a) - (d) correspond to step (a) - (d) of a method according the first aspect of the present invention. Accordingly, the preferred aspect of steps (a) - (d) are preferably those of steps (a) - (d) of a method according to the first aspect of the present invention.

In step (e), the immune therapy composition is adapted to increase the efficacy of the first immune therapy composition.

Any component of the immune therapy composition can be varied to improve the composition; e.g. the concentration, but also the nature of a compound can be varied. In this respect, the concentration of an antigen or adjuvant may be increased or decreased or another more appropriate adjuvant of antigen may be used, or an adjuvant or antigen may be added or removed. After design and production of a second immune therapy composition, a method according to the first and second aspect of the invention may be repeated. This entire process may then again be repeated once or multiple times to optimize a vaccine composition.

In a method according to the present invention for the preparation of an immune therapy composition the preparation of an immune therapy composition, a population of immune therapy-induced T-cells may comprise CD4+ T-cells. Preferably, the population comprises at least 10%, 20%, 30%, 40%, 50%, 60%, 70%, 80%, 90%, 95%, or at least 99% CD4+ T-cells of the total amount of cells in the population.

In a method according to the invention for the preparation of an immune therapy composition, a population of immune therapy-induced T-cells may comprise CD8+ T-cells. Preferably, the population comprises at least 10%, 20%, 30%, 40%, 50%, 60%, 70%, 80%, 90%, 95%, or at least 99% CD8+ T-cells of the total amount of cells in the population.

In a method according to the present invention for the preparation of an immune therapy composition, a population of immune therapy-induced T-cells may comprise both CD4+ T-cells and CD8+ T-cells. Preferably, the population comprises at least 10%, 20%, 30%, 40%, 50%, 60%, 70%, 80%, 90%, 95%, or at least 99% of both CD4+ T-cells and CD8+ T-cells of the total amount of cells in the population.

In a method according to the present invention for the preparation of an immune therapy composition, the immune therapy composition is may be a vaccine composition. The immune therapy composition and vaccine composition are preferably one according to the first aspect of the present invention.

In a third aspect, the present invention provides an immune composition obtainable by a method according to the second aspect of the present invention. Preferably, the immune composition is an immune composition obtained by a method according to the second aspect of the present invention.

An immune therapy composition according to this aspect of the present invention can conveniently be used in a method according to the invention.

In a fourth aspect, the present invention relates to a method of treatment of an individual with a immune therapy composition comprising:
a. administering an immune therapy composition to an individual,
b. performing a method according to the first aspect of the present invention, and optionally
c. adapting the immune therapy composition to the individuals needs.

Preferably, the immune composition is an immune composition as described in the first aspect of the present invention, more preferably, the immune composition is a vaccine composition as described in the first aspect of the present invention.

Step (b) corresponds to a method according to the first aspect of the present invention. Accordingly, the preferred features of step (b) are preferably those of a method according to the first aspect of the present invention.

Step (c) corresponds to step (e) of a method of the second aspect of the present invention. Accordingly, the preferred features of step (c) are preferably those of step (e) of a method of the second aspect of the invention.

An immune therapy composition may in any embodiment of the present invention be administered to an individual using any way available to the person skilled in the art. Ways of administration are known and customary in the art and for instance described in Remington; The Science and Practice of Pharmacy, 21st Edition 2005, University of Sciences in Philadelphia. Immune therapy compositions according to the invention are preferably formulated to be suitable for intravenous or subcutaneous, or intramuscular administration, although other administration routes can be envisaged, such as mucosal administration or intradermal and/or intracutaneous administration, e.g. by injection.

Prevention, treatment and delay of a disease or condition is preferably defined as an effect that ameliorates the situation of an individual. When the disease or condition is cancer, prevention, treatment and delay is preferably defined as an anti-tumor effect on a tumor. The tumor may be any tumor related to any cancer as defined earlier herein. An anti-tumor effect is preferably identified as:
- an activation or an induction of the systemic immune system: detectable and/or an increase in antigen specific activated CD4⁺ or CD8⁺ T-cells in peripheral blood or an increase thereof or of the cytokines produced by these T-cells after at least one week of treatment and/or
- an inhibition of proliferation of tumor cells or a detectable decrease of proliferation of tumor cells or a decrease in cell viability of tumor cells, which is equivalent to a decrease in tumor cell survival and/or
- an induction or increased induction of tumor cell death and/or
- an inhibition or prevention or delay of the increase of a tumor weight or growth and/or
- a prevention or delay in occurrence of metastases and/or of tumor cell migration, and/or
- a prevention of tumor recurrence and/or
- a prolongation of patient survival of at least one month, several months or more (compared to those not treated or treated with a control or compared with the subject at the onset of the treatment).

In the context of the invention, a patient may survive and may be considered as being disease free. Alternatively, the disease or condition may have been stopped or delayed or regressed. A significant increase of tumor-specific activated CD4⁺ or CD8⁺ cells in peripheral blood after at least one week of treatment may be of at least 5%, 10%, 20%, 30% or more. An inhibition of the proliferation of tumor cells may be at least 20%, 30%, 40%, 50%, 55%, 60%, 65%, 70% or 75%, or more. An induction of tumor cell death may be at least 1%, 5%, 10%, 15%, 20%, 25%, or more. Tumor growth may be inhibited at least 5%, 10%, 20%, 30%, 40%, 50%, 55%, 60%, 65%, 70% or 75%, or more. In certain embodiments, tumor weight increase may be inhibited at least 20%, 30%, 40%, 50%, 55%, 60%, 65%, 70% or 75%, or more. In certain embodiments, tumor growth may be delayed at least one week, one month, two months or more. A delay in occurrence of metastases and/or of tumor cell migration may be a delay of at least one week, one month, several months, one year or longer.

In each embodiment wherein the effect of an immune therapy composition according to the invention is quantified, the assay may be carried out by comparison to a subject not treated or to the same subject before treatment. A tumor can be a solid tumor or a non-solid tumor such as lymphoma. A tumor can be hormone-sensitive or hormone resistant tumors.

The invention further relates to a method of treatment of an individual with an immune therapy composition comprising administering an immune therapy composition according to the third aspect of the present invention to an individual.

Preferably, the immune composition is an immune composition as described in the first aspect of the present invention, more preferably, the immune composition is a vaccine composition as described in the first aspect of the present invention.

In a fifth aspect the present invention provides an immune therapy composition for treatment, prevention, reduction or delay of a disease or condition in an individual, wherein the immune therapy composition is administered to the individual, wherein subsequently a method according to the first aspect of the invention is performed and wherein subsequently the immune therapy composition is adapted to the individuals needs to obtain a second immune therapy composition.

Preferably, the immune composition is an immune composition as described in the first aspect of the present invention, more preferably, the immune composition is a vaccine composition as described in the first aspect of the present invention. The second immune composition obtained can conveniently be used in a method of treatment according to the fourth aspect of the present invention or can be for use for treatment, prevention, reduction or delay of a disease or condition in an individual.

The invention further relates to the use of an immune therapy composition for the preparation of a medicament, preferably for treatment, prevention, reduction or delay of a disease or condition in an individual wherein the immune therapy composition is administered to the individual, wherein subsequently a method according to the first aspect of the invention is performed and wherein subsequently the immune therapy composition is adapted to the individuals needs to obtain a second immune therapy composition.

Preferably, the immune composition is an immune composition as described in the first aspect of the present invention, more preferably, the immune composition is a vaccine composition as described in the first aspect of the present invention. The second immune composition obtained can conveniently be used in a method of treatment according to the fourth aspect of the present invention or can be for use for treatment, prevention, reduction or delay of a disease or condition in an individual.

The preferred features of this aspect of the invention are preferably those features of the fourth aspect of the invention, said fourth aspect relating to a method of treatments.

In a sixth aspect, the present invention further provides the use of at least one parameter selected from the group consisting of:
a. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+ in a population comprising immune therapy-induced T-cells,
b. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+ that are CD127- in a population comprising immune therapy-induced T-cells,
c. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+ that are CD62L- in a population comprising immune therapy-induced T-cells,
d. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+ that are CD127- and are CD62L- in a population comprising immune therapy-induced T-cells,
e. the frequency of at least one of antigen specific IFNgamma producing T-cells, antigen specific TNFalpha producing T-cells and antigen specific IL2 producing T-cells, preferably of antigen specific IFNgamma producing T-cells producing T-cells and antigen specific TNFalpha producing T-cells, more preferably of antigen specific IFNgamma producing T-cells, antigen specific TNFalpha producing T-cells and antigen specific IL2 producing T-cells, more preferably of antigen specific polyfunctional T-cells in a population comprising immune therapy-induced T-cells, and
f. the frequency of antigen specific immune therapy-induced T-cells in a population comprising immune therapy-induced T-cells,
for measuring the efficacy of an immune therapy composition in an individual. The use of a parameter according to this aspect of the invention is preferably as described in the first aspect of the invention.

Said use for measuring the efficacy of an immune therapy composition in an individual can be performed *in vivo, ex vivo* or *in vitro.*

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one". The word "about" or "approximately" when used in association with a numerical value (e.g. about 10) preferably means that the value may be the given value (of 10) more or less 0.1 % of the value.

All patent and literature references cited in the present specification are hereby incorporated by reference in their entirety.

Unless otherwise indicated each embodiment as described herein may be combined with another embodiment as described herein.

The embodiments as described herein may be performed *in vivo, ex vivo* or *in vitro.* In the embodiments of the present invention set out herein, killer cell lectin-like receptor G1 (KLRG1) is preferably used; in specific embodiments, KLRG1 may be substituted by another appropriate marker such as Granzyme B and Fas ligand (CD95L).

### FIGURE LEGENDS

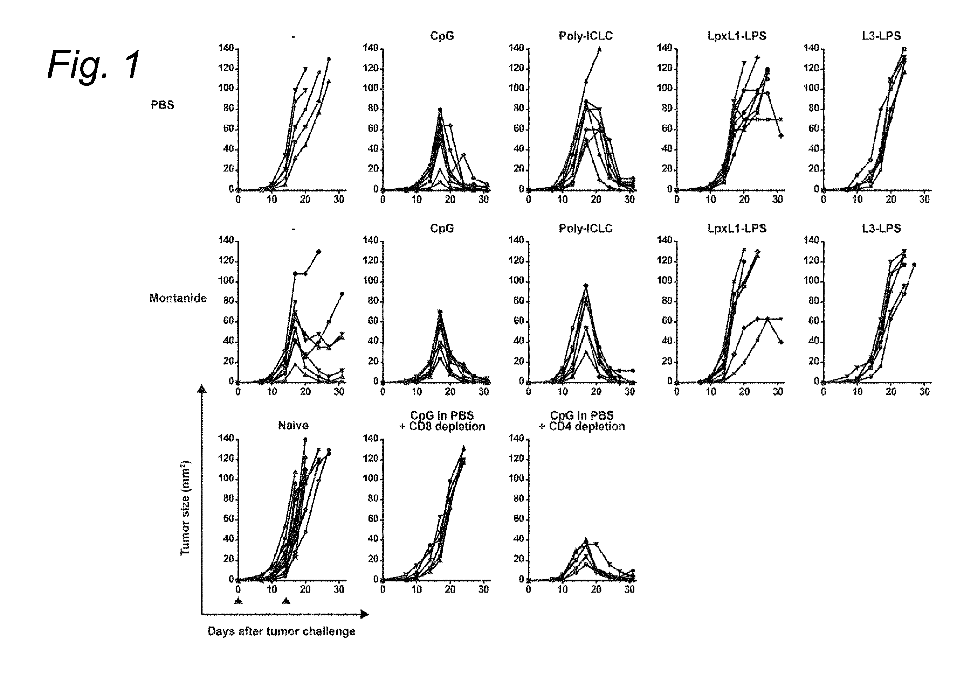

**Figure 1****. Vaccine-mediated tumor regression depends on induction of CD8+ T cell responses.**
   Wild-type C57BL/6 mice were injected subcutaneously in the flank with 1E+05 HPV16+ TC-1 tumor cells. Ten days later, when tumors were palpable, mice were left untreated (naive) or were treated with vaccine formulations containing synthetic long HPV16 E743-77 peptide with or without addition of CpG, Poly-ICLC, LpxL1-LPS or L3-LPS, dissolved in either PBS or Montanide provided in the other flank. Booster vaccination was provided 14 days after the first vaccination. CD8 or CD4 depleting antibodies were provided once a week starting at the day of vaccination to mice that received peptide/CpG vaccination. Shown is the TC-1 tumor outgrowth measured two-dimensionally (mm2) after tumor challenge. Each line represents a single mouse and 5-15 mice per group were used.

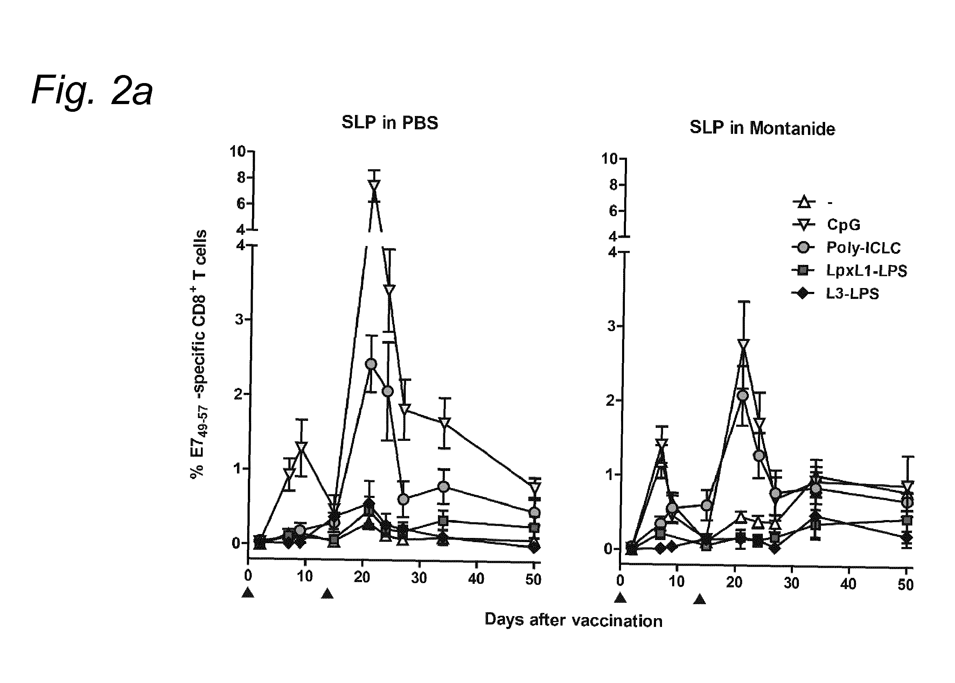

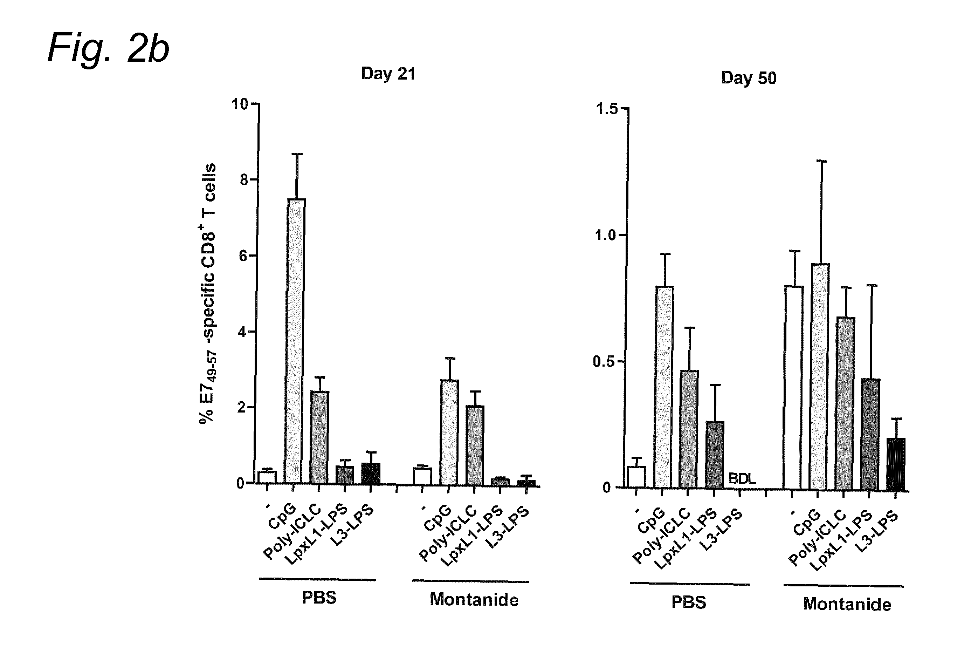

**Figure 2****. Vaccine formulations inducing high frequencies of antigen-specific CD8+ T cells correlate with efficacy.**
   Naive C57BL/6 mice (non-tumor bearing) were vaccinated twice on day 0 and 14 with different vaccine formulations containing HPV16 E7₄₃₋₇₇ long peptide with or without TLR agonists (CpG, Poly-ICLC, LpxL1-LPS or L3-LPS) dissolved in either PBS or Montanide.
   (A) Graphs show the number of E7-specific CD8⁺ T cells (± SEM) determined longitudinally (until day 50 post-vaccination) in the blood by MHC class I tetramers.
   (B) Bar graphs show the mean number of E7-specific CD8⁺ T cells (+ SEM) on day 21 and day 50 after the first vaccination (day 7 and day 36 after second vaccination). Each vaccinated group represents 5-10 mice. Experiments were performed twice with similar results. * *p* < 0.05, *** *p* < 0.0005 compared with no TLR adjuvant (Student t-test).

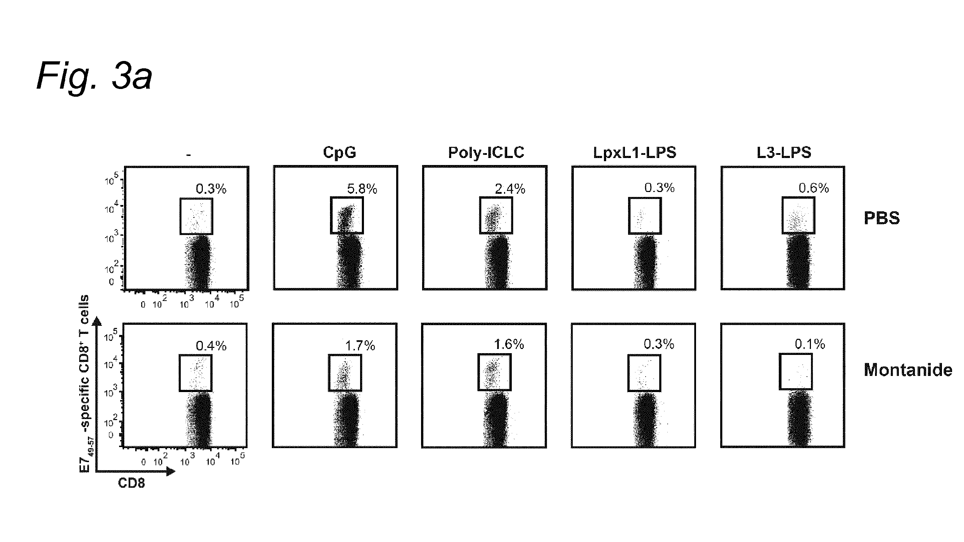

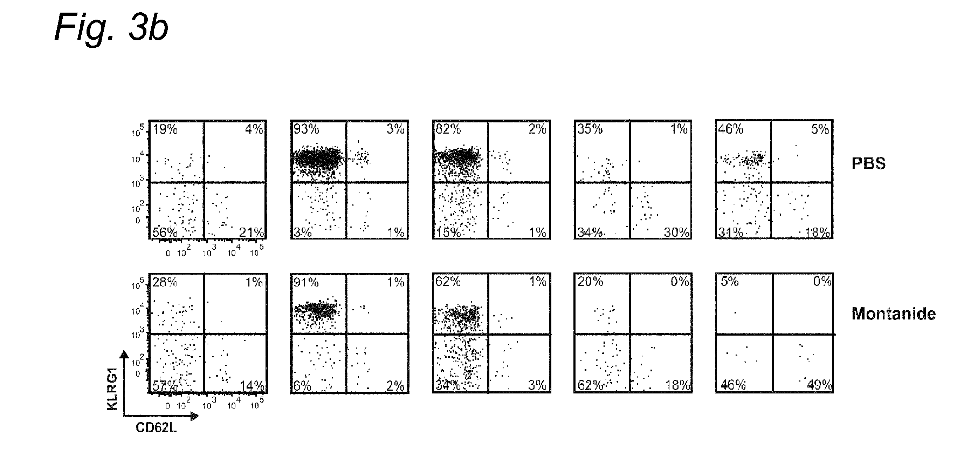

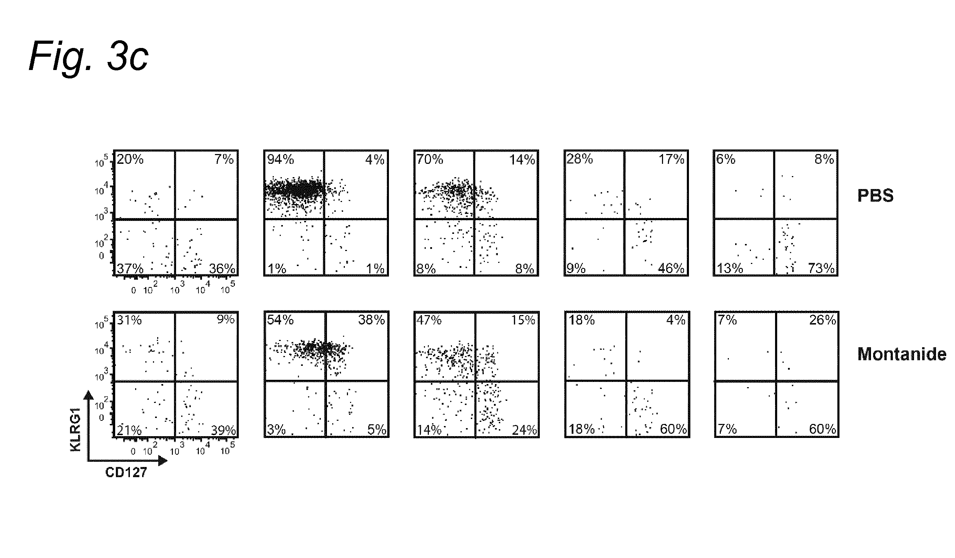

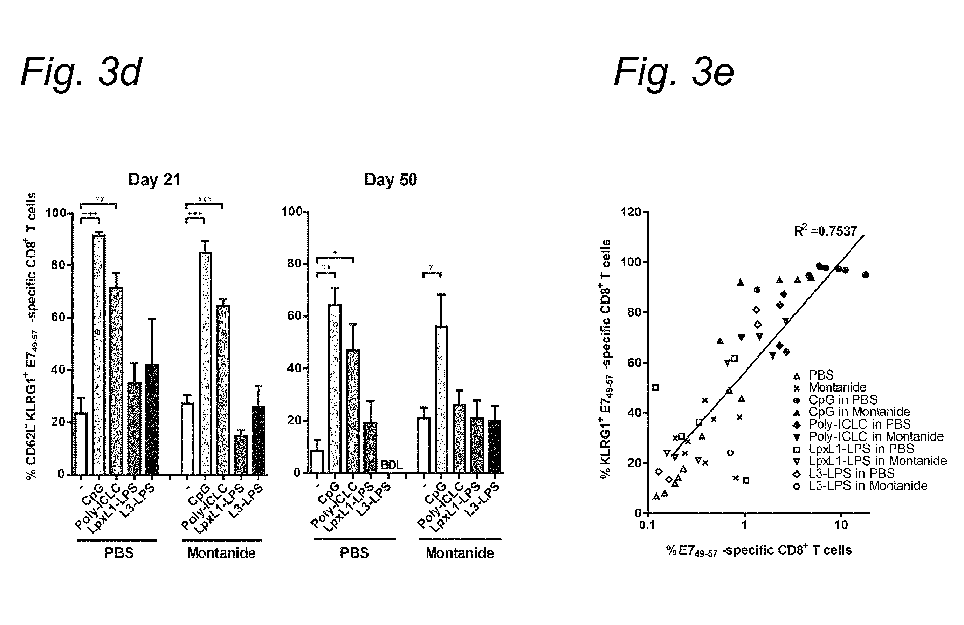

**Figure 3****. Vaccine formulations inducing formation of CD62L-/KLRG1⁺ effector-memory CD8⁺ T cells correlate with their therapeutic efficacy.**
   Naive C57BL/6 mice (non-tumor bearing) were vaccinated twice on day 0 and 14 with different vaccine formulations containing HPV16 E7₄₃₋₇₇ long peptide with or without TLR ligands (CpG, Poly-ICLC, LpxL1-LPS or L3-LPS) dissolved in either PBS or Montanide.
   **(A)** Shown are representative fluorescent intensity plots of E7-specific CD8⁺ T cell responses in blood of vaccinated mice at day 21 after the first vaccination (day 7 after the second vaccination) with the indicated vaccine formulations dissolved in PBS. Within this population of E7-specific CD8⁺ T cells the cell surface expression of
   **(B)** CD62L *versus* KLRG1 and
   **(C)** CD127 *versus* KLRG1 is depicted.
   **(D)** Bar graphs indicate the mean percentage of CD62L⁻KLRG1⁺ cells (+ SEM) within the E7-specific CD8⁺ T cell population at day 21 and day 50 after the first vaccination. Each bar represents 4 to 5 mice. At day 50 the E7-specific CD8⁺ T cell response in mice vaccinated with peptide and L3-LPS in PBS was below detection limit (BDL).
   **(E)** The percentage of CD62L⁻KLRG1⁺ CD8⁺ T cells correlates with the frequency of the total E7-specific CD8⁺ T cell population. Each symbol represents the E7-specific CD8⁺ T cell response in blood of an individual mouse on day 21 after the first vaccination. Experiments were performed twice with similar results. * *p* < 0.05, *** p* < 0.005, **** p* < 0.0005 compared with no TLR adjuvant (Student t-test).

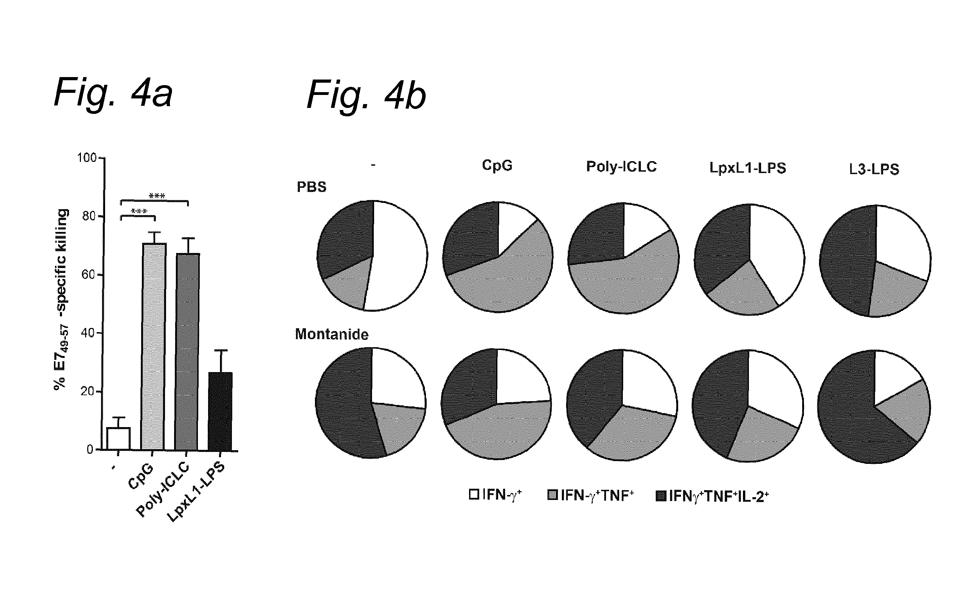

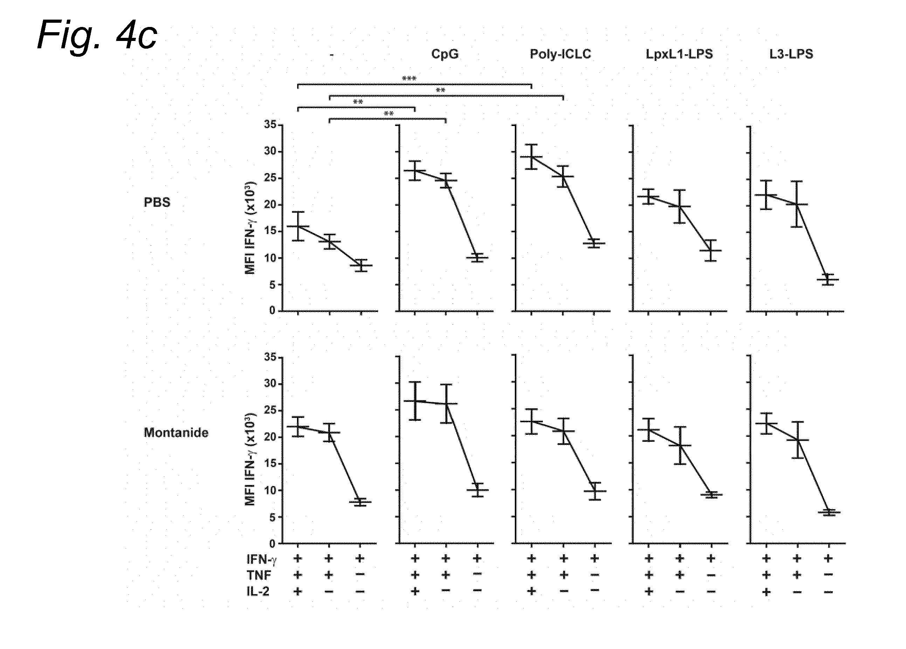

**Figure 4****. Simultaneous and high production of IFN-γ and TNF is a functional characteristic of effective effector-memory CD8⁺ T cells.**
   Naive C57BL/6 mice (non-tumor bearing) were vaccinated twice on day 0 and 14 with different vaccine formulations containing HPV16 E7₄₃₋₇₇ long peptide with or without TLR ligands (CpG, Poly-ICLC, LpxL1-LPS or L3-LPS) dissolved in either PBS or Montanide.
   **(A)** In vivo cytotoxicity assay. After loading CD45.1⁺ splenocytes with E7₄₉₋₅₇ or control peptide, cells were labeled with either a high dose or a low dose of CFSE. One day after injection of these target cells in vaccinated recipient mice, spleens were harvested and specific killing was calculated. Shown are the mean percentages (+ SEM) of E7-specific killing after the indicated vaccination formulations dissolved in PBS (n=4 to 8 mice).
   **(B)** Intracellular cytokine production of splenic E7-specific CD8⁺ T cells was determined after 5 h restimulation with HPV16 E7⁴⁹⁻⁵⁷ peptide at day 50 after the first vaccination. Pie charts show the proportion of triple (IFN-γ/TNF/IL-2), double (IFN-γ/TNF) and single (IFN-γ) cytokine producing cells.
   **(C)** Graphs show the mean fluorescence intensity (MFI) (± SEM) of IFN-γ expression in the triple, double and single IFN-γ producing cells. Results of 4 to 5 mice are shown. Experiments were performed twice with similar results. *** p < 0.0005 compared with no TLR adjuvant (Student t-test).

### SEQUENCES

**Table 1. Sequences as set forth in the Sequence Listing**

| SEQ ID NO: | SEQ type | Gene product |
|---|---|---|
| 1 | DNA | KLRG1 |
| 2 | cDNA | KLRG1 |
| 3 | PRT | KLRG1 splice isoform 189 amino acids |
| 4 | PRT | KLRG1 195 amino acids |
| 5 | PRT | HPV 16 E7₄₃₋₇₇ peptide |
| 6 | PRT | HPV16 E7₄₉₋₅₇ peptide |

The present invention is further described by the following examples which should not be construed as limiting the scope of the invention.

Unless stated otherwise, the practice of the invention will employ standard conventional methods of molecular biology, virology, microbiology or biochemistry. Such techniques are described in Sambrook et al. (1989) Molecular Cloning, A Laboratory Manual (2nd edition), Cold Spring Harbor Laboratory, Cold Spring Harbor Laboratory Press; in Sambrook and Russell (2001) Molecular Cloning: A Laboratory Manual, Third Edition, Cold Spring Harbor Laboratory Press, NY; in Volumes 1 and 2 of Ausubel et al. (1994) Current Protocols in Molecular Biology, Current Protocols, USA; and in Volumes I and II of Brown (1998) Molecular Biology LabFax, Second Edition, Academic Press (UK); Oligonucleotide Synthesis (N. Gait editor); Nucleic Acid Hybridization (Hames and Higgins, eds.).

### EXAMPLES

### Introduction

In this study, we assessed whether CD8⁺ T cell responses elicited by various vaccine formulations in non-tumor settings could be useful in defining immune correlates for tumor eradication. These vaccine formulations include the use of Toll like receptor (TLR) agonists, known to be important initiators of innate and adaptive immune responses. Our results show that vaccine-induced effector-memory T cell responses in a tumor-free setting, defined by a CD62L⁻/KLRG1⁺ phenotype and simultaneous secretion of IFNgamma and TNFalpha, predicts best the degree of therapeutic vaccine efficacy against established subcutaneous tumors. The premise that the characteristics of vaccine-induced T cell responses in tumor-free animals can forecast the therapeutic efficacy during tumor therapy implies that characterization of CD8⁺ T cell responses is critical for rational design of (therapeutic) vaccines against cancer.

### Material and Methods

### Mice

Female C57BL/6 (H-2^{b}) and congenic B6.SJL (CD45.1, Ly5.1) mice were purchased from Charles River (France) and maintained in in the central animal facility of Leiden University Medical Center (LUMC). All mice were housed in specific pathogen-free conditions and used at 8-10 weeks of age. All animal experiments were approved by the Animal Experiments Committee of the LUMC and performed according to the guide to animal experimentation set by the LUMC and to the Dutch Experiments on Animals Act that serves the implementation of 'Guidelines on the protection of experimental animals' by the Council of Europe.

### Vaccination

Long HPV16 E7₄₃₋₇₇ peptide (GQAEP**DRAHYNI**VTFCCKCDSTLRLCVQSTHVDIR) (SEQ ID NO: 4), covering both the CTL epitope (indicated in bold letters) and the Tₕₑₗₚₑᵣ epitope (underlined) was mixed in PBS or Montanide ISA 51 VG (Seppic) in a total volume of 200 µl per injection. Per mouse 150 µg of this 35-mer synthetic long peptide (SLP) was used with or without addition of an adjuvant. Adjuvants used were the following; CpG (ODN1826; 20 µg per mouse, purchased from InvivoGen), Poly-ICLC (Hiltonol; 50 µg per mouse, kindly provided by Oncovir), LpxL1-LPS (Imsavac-L; 10 µg per mouse) and L3-LPS (20 µg per mouse). LpxL1-LPS and L3-LPS were received via the National Institute of Public Health and the Environment, The Netherlands). Vaccine formulations in Montanide were mixed in a 1:1 ratio with adjuvant and peptide dissolved in PBS. All vaccinations were prepared on the day of injection and given subcutaneously.

### Tumor regression experiment

TC-1 tumor cells are derived from primary lung epithelial cells of C57BL/6 mice and co-transformed with HPV-16 E6 and E7 and c-Ha-Ras oncogenes (Lin et al, 1996). These cells were cultured at 37°C with 5% CO₂ in IMDM containing 8% FCS (Greiner), 2 mM Glutamin and 100 IU/ml penicillin in the presence of 400 µg/ml Geneticin (G418; Gibco), non essential amino acids (1:100; Gibco) and 1 mM Sodium Pyruvate (Gibco).
On day 0, mice were challenged by subcutaneous (s.c.) injection in the flank with 1 x 10⁵ TC-1 tumor cells in a total volume of 200 µl PBS. On day 10 after tumor challenge, when tumors were palpable (9 mm²) mice were split into groups with similar tumor size and vaccinated as described above in the contra lateral flank. As a control naive mice were injected with tumor cells only. Twice a week the tumor sizes were measured two dimensionally with calipers to a maximum of 150 mm² after which the mice were sacrificed for ethical reasons.

### In vivo T cell depletion

For in vivo CD4⁺ and CD8⁺ T cell depletion, we injected mice intraperitoneally (i.p.) with 100 microgram of the monoclonal antibodies GK1.5 and 2.43, respectively, on days 0, 7, 11 and 14 after vaccinations. Monoclonal antibodies were prepared and purified as described(11).

### Flow cytometry.

Cell surface staining was performed on freshly prepared peripheral blood mononuclear cells and splenocytes after red blood cell lysis. Cells were surface stained for 30 minutes with allophycocyanin-labeled H-2D^{b} E7₄₉₋₅₇ tetramer (produced in house) and fluorescently labeled antibodies specific for CD8alpha, CD127 (IL-7Ralpha), CD62L and KLRG1 (purchased from BD and ebioscience) in staining buffer (PBS containing 1% FCS and 0.05% sodium azide). 7-AAD was used for dead cell exclusion. Flow cytometric intracellular cytokine analysis of peripheral blood mononuclear cells and splenocytes was performed after 5 hour stimulation with the HPV16 E7₄₉₋₅₇ peptide (5 µg/ml= mM) in presence of Brefeldin A (2 µg/ml). After cell surface staining with CD8 antibodies, cells were fixed with Cytofix/Cytoperm solution (BD), and permeabilized with Perm/Wash buffer. Subsequently, cells were stained for 30 minutes at 4°C with fluorescently labeled antibodies against IFNgamma, TNF and IL-2. Samples were acquired with a BD LSRII Flowcytometer and results were analyzed using FlowJo software (Treestar).

### In vivo cytotoxicity

Cells from mice vaccinated with HPV16 E7₄₃₋₇₇ peptide in combination with different adjuvants either in PBS or Montanide ISA 51 were used as effectors in this assay. Splenocytes from naive congenic CD45.1⁺ mice were used as target cells. These cells were counted and split into two equal parts. One part was pulsed for one hour with the specific HPV16 E7₄₉₋₅₇ (RAHYMVTF) (SEQ ID NO: 5) peptide and one part with unspecific control peptide (Adenovirus type 5 E1A₂₃₄₋₂₄₃). After washing the cells, E7-peptide loaded cells were fluorescently labeled with 5 µM CFSE while control peptide cells were labeled with 0.5 µM CFSE. Target cells were injected intravenously (i.v.) in 200 µl PBS in a 1:1 ratio in recipient mice. One day after the adoptive transfer, spleens were harvested, a single cell suspension was made and red blood cell lysis was performed. Flow cytometry was performed on splenocytes and specific lysis was calculated according to the following formula: (1-[(CFSE^{hi}/CFSE^{lo})_{vaccinated} /(CFSE^{hi}/CFSE^{lo})_{naïve}]) * 100%.

### Statistical analyses

We assessed the significance of differences in the magnitude, phenotypical and functional properties of CD8⁺ T cells by two-tailed Student *t*-tests. *p* values < 0.05 were considered significant. Correlations were analyzed with linear regression.

### Results

### Vaccine-mediated tumor regression depends on induction of CD8⁺ T cell responses

To define immune correlates of vaccine-mediated protection against tumors *in vivo* we tested multiple therapeutic vaccine formulations in a preclinical model of human papillomavirus (HPV)16-induced cervical cancer (Zwaveling et al, 2002) We examined vaccine formulations containing the long peptide E7₄₃₋₇₇ mixed with the adjuvants CpG (TLR9 ligand), polyinosinic-polycytidylic acid stabilized by lysine and carboxymethylcellulose (Poly-ICLC, TLR3 ligand) or different forms of LPS (TLR4 ligand), i.e. *N. meningitidis* wild-type L3-LPS and mutant penta-acylated LpxL1-LPS (Fransen et al, 2007), for their efficacy to restrain the outgrowth of established transplanted HPV16⁺ TC-1 tumors expressing the E7 oncogene. These vaccine formulations were dissolved in either PBS or Montanide, a clinically approved mineral oil that causes a gradual release of antigen (Kenter et al, 2009; Fransen et al, 2011). On day 10 after TC-1 tumor inoculation, when tumors were palpable (-9 mm²), mice were vaccinated twice with the different vaccine formulations with a two week interval.
All non-vaccinated mice developed tumors of >100 mm² within 25 days after tumor inoculation (Fig. 1). Vaccination with peptide in PBS had no inhibitory effect on tumor outgrowth, while peptide in Montanide delayed or inhibited outgrowth of TC-1 tumors, indicating that the depot function of Montanide considerably improves tumor eradication. Notably, the addition of either CpG or Poly-ICLC in PBS as well as in Montanide induced substantial tumor regression compared to peptide/PBS vaccination, suggesting that a depot effect is less important when TLR3 or TLR9 stimulation is provided. On the other hand, LpxL1-LPS and L3-LPS containing formulations induced only a marginal inhibitory effect on tumor growth compared to CpG and Poly-ICLC (Fig. 1). The requirement for CD8⁺ and/or CD4⁺ T cells in mediating tumor growth inhibition was assessed experimentally by depletion of these subsets at the time of peptide/CpG vaccination. The lack of CD8⁺ T cells but not of CD4⁺ T cells abolished the vaccine-induced tumor regression (Fig. 1). Together these results indicate a differential impact of TLR agonists containing vaccine formulations on tumor progression in a CD8⁺ T cell dependent fashion.

### Vaccine formulations inducing high frequencies of antigen-specific CD8⁺ T cells correlate with their therapeutic efficacy

Based on the importance of CD8⁺ T cells for eradication of subcutaneous tumors, we decided to test whether vaccinations in a tumor free setting could predict the efficacy of these vaccines in therapeutic situations by determining the characteristics of the CD8⁺ T cell responses such as magnitude and quality. To this end, wild-type (non-tumor bearing) mice were vaccinated with the same vaccine formulations and schedule as describe aforementioned. The magnitude of the peptide-specific CD8⁺ T cell response was monitored longitudinally in blood with H-2D^{b} E7₄₉₋₅₇ tetramers (Fig. 2A). One week after the first vaccination, the mixture of peptide in Montanide but not in PBS elicited detectable E7-specific CD8⁺ T cell responses (∼1%). Addition of the adjuvant CpG increased the E7-specific response when used in a mixture with PBS (∼1%) but similar percentages as peptide alone were found with Montanide. The adjuvants Poly-ICLC, LpxL1-LPS or L3-LPS elicited no substantial increase in E7-specific cells, either dissolved in PBS or Montanide (Fig. 2A and 2B). Seven days after the second (boost) vaccination (day 21 post first vaccination), the E7-specific T cell responses were significantly higher in mice that received peptide vaccines containing CpG, dissolved in either PBS or Montanide, compared to peptide only vaccines. Also, vaccine formulations with poly-ICLC, dissolved in PBS and Montanide, elicited high frequencies of E7-specific CD8⁺ T cells. Importantly, these CpG and Poly-ICLC containing vaccine formulations had profound efficacy against established TC-1 tumors (Fig. 1). In contrast, presence of the adjuvants LpxL1-LPS or L3-LPS, which were not effective in reducing tumor outgrowth, did not markedly increase the frequency of antigen-specific CD8⁺ T cells after the booster vaccination (Fig. 2A and 2B). Several weeks later (day 50 after the first immunization), the percentage of E7-specific CD8⁺ T cells elicited by CpG and Poly-ICLC dissolved in PBS but not in Montanide was still significantly higher in the vaccinated group. These results indicate that the frequency of vaccine-specific CD8⁺ T cells after booster vaccination in a tumor-free setting correlates with the therapeutic vaccine efficacy against established tumors.

### Vaccine formulations inducing formation of CD62L⁻/KLRG1⁺ effector-memory CD8⁺ T cells correlate with their therapeutic efficacy

Distinct subsets of CD8⁺ T cells expand upon antigen re-encounter (Sallusto et al, 2004; Huster et al, 2006). Effector T cells dominate the expansion phase, migrate to peripheral organs, and display immediate effector function. Effector-memory T cells preferentially home to peripheral tissues and respond to antigen encounter with immediate effector functions while central-memory T cells home to lymphoid organs and can strongly expand upon antigen re-encounter (Sallusto et al, 2004; Huster et al, 2006). Here we determined by polychromatic flow cytometry (Perfetto et al, 2004) the formation of effector and memory T cell populations within the vaccine-induced E7-specific CD8⁺ T cell population based on the cell-surface expression of lymphocyte homing receptor CD62L, killer cell lectin-like receptor G1 (KLRG1), and IL-7 receptor alpha (CD127) (Fig. 3A, B). After the booster vaccination the percentages of effector/effector-memory cells, defined by CD62L⁻KLRG1⁺, were significantly higher in the effective adjuvant groups only (i.e. containing CpG and Poly-ICLC) (Fig. 3B and 3D). The cell surface marker CD127 (IL-7R□) was, albeit more than CD62L, lowly expressed on the KLRG-1⁺ CD8⁺ T cells (Fig. 3C and Supplemental Fig. 1B). Thus, like the frequency of vaccine-specific T cells, the cell surface phenotype (i.e. CD62L-/KLRG1⁺) of these cells after vaccination in tumor-free mice is predictive for therapeutic vaccine efficacy against established tumors. Moreover, since a positive correlation (correlation coefficient R²=0.7537) exists between the magnitude of the E7-specific T cell response and the percentage of CD62L⁻KLRG1⁺ cells within this population (Fig. 3E), either the percentage of antigen-specific CD8⁺ T cells or the CD62L⁻KLRG-1⁺ phenotype of these cells can be used as a predictor for effective therapeutic tumor vaccines. At later time points post-vaccination, the CD62L-KLRG-1⁺ cell surface expression was also significantly higher on antigen-specific CD8⁺ T cells elicited by vaccine formulations containing CpG (dissolved in either PBS or Montanide) and Poly-ICLC in PBS but not in Montanide (Fig. 3D). Together, these results indicate that at early times after vaccine boosting both the frequency and phenotype of vaccine-induced CD8⁺ T cells can predict effective vaccine formulations but at later time points post-booster the predictive authority of T cell frequency and phenotype induced by vaccine formulations dissolved in Montanide wanes.

### Simultaneous and high production of IFNalpha and TNF is a functional characteristic of effective effector-memory CD8⁺ T cells

To determine the functional killing capacity of the vaccine-induced effector/effector-memory T cell pools we *performed in vivo* cytotoxicity assays (Fig. 4A). Vaccination with either CpG or Poly-ICLC resulted in strong cytolytic activity (i.e. ∼70%), while the killing of target cells in mice immunized with LpxL1-LPS or peptide alone are low (<30%) (Fig. 4A). Besides cytolytic activity the capacity to produce effector cytokines such as IFNalpha is a key mechanism for tumor eradication by CD8⁺ T cells ()Dunne et al, 2005) At day 50 after post-vaccination we measured cytokine production by performing polychromatic intracellular cytokine staining (Fig. 4B). In mice immunized with synthetic long peptides in PBS, the majority of the IFN-γ⁺ cells (∼50%) were single producers, which are known to have a poor capacity to develop into memory cells (Wu et al, 2002). Addition of the adjuvants CpG and poly-ICLC resulted in a relative decrease of single producers and a related increase in the frequency of IFNgamma/TNF double producing cells (Fig. 4A). Delivery of vaccines in Montanide instead of PBS resulted in an increase in double producers and a concomitant decrease in triple producing CD8⁺ T cells of the CpG and Poly-ICLC vaccine groups as compared to the Montanide only group (Fig. 4A). Both the LpxL1-LPS and L3-LPS adjuvants did not induce increased percentages of double-producers either dissolved in PBS or Montanide (Fig. 4B). Thus, the production of simultaneous IFNgamma/TNF but not of triple IFNgamma/TNF/IL-2 or single IFNgamma is a functional capacity of effective vaccine-induced T cells.
Consistent with the hierarchy of the vaccine efficacy of the formulations dissolved in PBS, the mean fluorescence intensity (MFI) of IFNgamma production within the E7-specific CD8⁺ T cells, reflecting the production of this cytokine on a per cell basis, within the triple (IFNgamma/TNF/IL-2) and double (IFNgamma/TNF) positive populations was the highest for the CpG and Poly-ICLC containing vaccines followed by LpxL1-LPS and L3-LPS. Of the vaccine formulations dissolved in Montanide only the CpG containing vaccine elicited a high IFNgamma MFI. None of the vaccine formulations dissolved in either PBS or Montanide elicited any difference in the IFN-□ MFI of the single IFNgamma producing CD8⁺ T cells, which was much lower as IFNgamma MFI of the triple and double producers (Fig. 4C). Thus, also the increased production of IFN-γ on a per cell basis is a functional characteristic of the effector-memory CD8⁺ T cells that are elicited by the vaccine formulations that are effective in a therapeutic setting.

### Discussion

In this study, we have delineated immune correlates for tumor eradication based on the frequency and heterogeneity of antigen-specific CD8⁺ T cell responses elicited by various vaccine formulations in tumor-free conditions. Vaccines containing the TLR ligands CpG (TLR9) and Poly-ICLC (TLR3) elicited increased frequencies of effector-memory cells (CD62L⁻/KLRG1⁺/IFNgamma⁺/TNF⁺) after vaccination in non-tumor settings, which correlated with superior effect of these vaccines on inhibiting tumor outgrowth when used as therapeutic vaccines. Furthermore our study indicates that induction of effector-memory CD8⁺ T cells is preferred over central-memory cells in case of effective regression of subcutaneous tumors. This is consistent with the fact that effector-memory T cells can migrate to or are already present in extra-lymphoid sites. In addition, effector-memory cells have compared to central-memory cells immediate effector function (Sallusto et al, 2004; Huster et al, 2006). Consistent with our findings are recent reports showing that vaccines eliciting persistent effector-memory T cell responses have an overall superior protective capacity against mucosal infection by chronic pathogenic viruses in comparison with vaccines that induce central-memory T cell responses (Hansen et al, 2009; Hansen et al, 2011). In contrast, ongoing systemic infections are usually better controlled by central-memory T cell responses than by effector-memory T cell responses (Wherry et al, 2003), presumably due to their better capacity to expand and to their preference for homing in spleen and lymph nodes. Although the peptide vaccines containing TLR3/9 agonists induced regression of established tumors, immune editing mechanisms (Schreiber et al, 2011) such as class I downmodulation can occur (Zwaveling et al, 2002). Strategies that specifically counterattack such immune evasion as well as therapies with tumor cell-apoptosis inducing therapies (e.g. chemotherapy and/or radiotherapy) (Lake et al, 2005; Formenti et al, 2009) may therefore be synergistic with peptide-based vaccines for tumor eradication. In addition, tumor-specific T cells may eventually display an exhausted phenotype and overcoming this (e.g. by PD-L1 blockade) can also improve therapeutic results (Kim et al, 2010; Baitsch et al, 2011)
In conclusion, our findings show that the efficacy of therapeutic vaccine formulations against cancer can be predicted based on characteristics of the antigen-specific T cell response that is elicited in healthy (non-tumor bearing) individuals. Further understanding of the mechanisms that influence the expansion and development of heterogeneous CD8⁺ T cell populations can be used to advance vaccine design. Our findings could therefore be valuable for improving and expediting the design of therapeutic vaccines against cancer.

### REFERENCE LIST

1. Kaech, S.M., E.J.Wherry, and R.Ahmed. 2002. Effector and memory T-cell differentiation: implications for vaccine development. Nat.Rev.Immunol. 2:251-262.
2. Arens, R. and S.P.Schoenberger. 2010. Plasticity in programming of effector and memory CD8 T-cell formation. Immunol.Rev. 235:190-205.
3. Melief, C.J. 2008. Cancer immunotherapy by dendritic cells. Immunity. 29:372-383.
4. Harari, A., V.Dutoit, C.Cellerai, P.A.Bart, R.A.Du Pasquier, and G.Pantaleo. 2006. Functional signatures of protective antiviral T-cell immunity in human virus infections. Immunol.Rev. 211:236-254.
5. Darrah, P.A., D.T.Patel, P.M.De Luca, R.W.Lindsay, D.F.Davey, B.J.Flynn, S.T.Hoff, P.Andersen, S.G.Reed, S.L.Morris, M.Roederer, and R.A.Seder. 2007. Multifunctional TH1 cells define a correlate of vaccine-mediated protection against Leishmania major. Nat.Med. 13:843-850.
6. Seder, R.A., P.A.Darrah, and M.Roederer. 2008. T-cell quality in memory and protection: implications for vaccine design. Nat.Rev.Immunol. 8:247-258.
7. Zwaveling, S., S.C.Ferreira Mota, J.Nouta, M.Johnson, G.B.Lipford, R.Offringa, S.H.van der Burg, and C.J.Melief. 2002. Established human papillomavirus type 16-expressing tumors are effectively eradicated following vaccination with long peptides. J.Immunol. 169:350-358.
8. Sallusto, F., J.Geginat, and A.Lanzavecchia. 2004. Central memory and effector memory T cell subsets: function, generation, and maintenance. Annu.Rev.Immunol. 22:745-763.
9. Huster, K.M., C.Stemberger, and D.H.Busch. 2006. Protective immunity towards intracellular pathogens. Curr.Opin.Immunol. 18:458-464.
10. Perfetto, S.P., P.K.Chattopadhyay, and M.Roederer. 2004. Seventeen-colour flow cytometry: unravelling the immune system. Nat.Rev.Immunol. 4:648-655.
11. Dunn, G.P., H.Ikeda, A.T.Bruce, C.Koebel, R.Uppaluri, J.Bui, R.Chan, M.Diamond, J.M.White, K.C.Sheehan, and R.D.Schreiber. 2005. Interferon-gamma and cancer immunoediting. Immunol.Res. 32:231-245.
12. Wu, C.Y., J.R.Kirman, M.J.Rotte, D.F.Davey, S.P.Perfetto, E.G.Rhee, B.L.Freidag, B.J.Hill, D.C.Douek, and R.A.Seder. 2002. Distinct lineages of T(H)1 cells have differential capacities for memory cell generation in vivo. Nat.Immunol. 3:852-858.
13. Johansen, P., T.Storni, L.Rettig, Z.Qiu, A.Der-Sarkissian, K.A.Smith, V.Manolova, K.S.Lang, G.Senti, B.Mullhaupt, T.Gerlach, R.F.Speck, A.Bot, and T.M.Kundig. 2008. Antigen kinetics determines immune reactivity. Proc.Natl.Acad.Sci.U.S.A 105:5189-5194.
14. Kenter, G.G., M.J.Welters, A.R.Valentijn, M.J.Lowik, Berends-van der Meer DM, A.P.Vloon, F.Essahsah, L.M.Fathers, R.Offringa, J.W.Drijfhout, A.R.Wafelman, J.Oostendorp, G.J.Fleuren, S.H.van der Burg, and C.J.Melief. 2009. Vaccination against HPV-16 oncoproteins for vulvar intraepithelial neoplasia. N.Engl.J.Med. 361:1838-1847.
15. Fransen, M.F., M.Sluijter, H.Morreau, R.Arens, and C.J.Melief. 2011. Local Activation of CD8 T Cells and Systemic Tumor Eradication without Toxicity via Slow Release and Local Delivery of Agonistic CD40 Antibody. Clin.Cancer Res. 17:2270-2280.
16. Schreiber, R.D., L.J.Old, and M.J.Smyth. 2011. Cancer immunoediting: integrating immunity's roles in cancer suppression and promotion. Science 331:1565-1570.
17. Lake, R.A. and B.W.Robinson. 2005. Immunotherapy and chemotherapy--a practical partnership. Nat.Rev.Cancer 5:397-405.
18. Formenti, S.C. and S.Demaria. 2009. Systemic effects of local radiotherapy. Lancet Oncol. 10:718-726.
19. Kim, P.S. and R.Ahmed. 2010. Features of responding T cells in cancer and chronic infection. Curr.Opin.Immunol. 22:223-230.
20. Baitsch, L., P.Baumgaertner, E.Devevre, S.K.Raghav, A.Legat, L.Barba, S.Wieckowski, H.Bouzourene, B.Deplancke, P.Romero, N.Rufer, and D.E.Speiser. 2011. Exhaustion of tumor-specific CD8 T cells in metastases from melanoma patients. J.Clin.Invest 121:2350-2360.
21. Hansen, S.G., C.Vieville, N.Whizin, L.Coyne-Johnson, D.C.Siess, D.D.Drummond, A.W.Legasse, M.K.Axthelm, K.Oswald, C.M.Trubey, M.Piatak, Jr., J.D.Lifson, J.A.Nelson, M.A.Jarvis, and L.J.Picker. 2009. Effector memory T cell responses are associated with protection of rhesus monkeys from mucosal simian immunodeficiency virus challenge. Nat.Med. 15:293-299.
22. Hansen, S.G., J.C.Ford, M.S.Lewis, A.B.Ventura, C.M.Hughes, L.Coyne-Johnson, N.Whizin, K.Oswald, R.Shoemaker, T.Swanson, A.W.Legasse, M.J.Chiuchiolo, C.L.Parks, M.K.Axthelm, J.A.Nelson, M.A.Jarvis, M.Piatak, Jr., J.D.Lifson, and L.J.Picker. 2011. Profound early control of highly pathogenic SIV by an effector memory T-cell vaccine. Nature 473:523-527.
23. Wherry, E.J., V.Teichgraber, T.C.Becker, D.Masopust, S.M.Kaech, R.Antia, U.H.von Andrian, and R.Ahmed. 2003. Lineage relationship and protective immunity of memory CD8 T cell subsets. Nat.Immunol. 4:225-234.
24. Fransen, F., C.J.Boog, J.P.van Putten, and P.van der Ley. 2007. Agonists of Toll-like receptors 3, 4, 7, and 9 are candidates for use as adjuvants in an outer membrane vaccine against Neisseria meningitidis serogroup B. Infect.Immun. 75:5939-5946.
25. Lin, K.Y., F.G.Guarnieri, K.F.Staveley-O'Carroll, H.I.Levitsky, J.T.August, D.M.Pardoll, and T.C.Wu. 1996. Treatment of established tumors with a novel vaccine that enhances major histocompatibility class II presentation of tumor antigen. Cancer Res. 56:21-26.
26. Arens, R., A.Loewendorf, M.J.Her, K.Schneider-Ohrum, G.R.Shellam, E.Janssen, C.F.Ware, S.P.Schoenberger, and C.A.Benedict. 2011. B7-mediated costimulation of CD4 T cells constrains cytomegalovirus persistence. J.Virol. 85:390-396.
27. Obar JJ, L. Lefrançois. 2010. Memory CD8+ T cell differentiation. Ann N Y Acad Sci. 1183: 251-66.
28. Kutscher, S. 2010. MVA-nef induces HIV-1-specific polyfunctional and proliferative T-cell responses revealed by the combination of short- and long-term immune assays. Gene Therapy 17, 1372-1383.
29. Snell, M. 2011. T-cell intrinsic effects of GITR and 4-1BB during viral infection and cancer immunotherapy. Immunological Reviews Vol. 244: 197-217.
30. Maecker HT. Multiparameter flow cytometry monitoring of T cell responses. Methods Mol Biol. 2009;485:375-91.
31. Lovelace P, Maecker HT. Multiparameter intracellular cytokine staining. Methods Mol Biol. 2011;699:165-78.
32. Foster B, Prussin C, Liu F, Whitmire JK, Whitton JL.Detection of intracellular cytokines by flow cytometry. Curr Protoc Immunol. 2007 Aug;Chapter 6:Unit 6.24.
33. Maecker HT, Rinfret A, D'Souza P, Darden J, Roig E, Landry C, Hayes P, Birungi J, Anzala O, Garcia M, Harari A, Frank I, Baydo R, Baker M, Holbrook J, Ottinger J, Lamoreaux L, Epling CL, Sinclair E, Suni MA, Punt K, Calarota S, El-Bahi S, Alter G, Maila H, Kuta E, Cox J, Gray C, Altfeld M, Nougarede N, Boyer J, Tussey L, Tobery T, Bredt B, Roederer M, Koup R, Maino VC, Weinhold K, Pantaleo G, Gilmour J, Horton H, Sekaly RP. Standardization of cytokine flow cytometry assays. BMC Immunol. 2005 Jun 24; 6:13.
34. Coffman, R.L., Sher, A., and Seder, R.A. (2010). Vaccine adjuvants: putting innate immunity to work. Immunity 29, 492-503.
35. Klinman, D.M. (2006). Adjuvant activity of CpG oligodeoxynucleotides. Int Rev Immunol. 25, 135-154.
36. Caskey, M., Lefebvre, F., Filali-Mouhim, A., Cameron, M.J., Goulet, J.P., Haddad, E.K., Breton, G., Trumpflieller, C., Pollak, S., Shimeliovich, I., Duque-Alarcon, A., Pan, L., Nelkenbaum, A., Salazar, A.M., Schlesinger, S.J., Steinman, R.M., Sékaly, R.P. (2011) Synthetic double-stranded RNA induces innate immune responses similar to a live viral vaccine in humans. J Exp Med. 208, 2357-2366.
37. Fransen, F., Boog, C.J., van Putten, J.P., van der Ley, P. (2007). Agonists of Toll-like receptors 3, 4, 7, and 9 are candidates for use as adjuvants in an outer membrane vaccine against Neisseria meningitidis serogroup B. Infect Immun. 75, 5939-5946.
38. Chiang, C.L., Kandalaft, L.E., Coukos, G. (2011). Adjuvants for enhancing the immunogenicity of whole tumor cell vaccines. Int Rev Immunol. 30, 150-182.

## Claims

1. An *ex vivo* method for measuring efficacy of an immune therapy composition in an individual, comprising:
a. providing a population of cells comprising T-cells from a sample obtained from an individual,
b. optionally isolating a population of antigen specific immune therapy-induced T-cells from the population obtained in step (a) and optionally measuring the frequency of antigen specific immune therapy-induced T-cells in the population of immune therapy-induced T-cells,
c. measuring in the population obtained in step (a) or (b) at least one of:
i. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+, and
ii. the frequency of at least one of antigen specific IFNgamma producing T-cells, antigen specific TNFalpha producing T-cells and antigen specific IL2 producing T-cells, preferably of antigen specific IFNgamma producing T-cells and antigen specific TNFalpha producing T-cells, more preferably of antigen specific IFNgamma producing T-cells, antigen specific TNFalpha producing T-cells and antigen specific IL2 producing T-cells, more preferably of antigen specific polyfunctional T-cells; and
d. correlating a parameter measured in (b) and/or (c) with the efficacy of the immune therapy composition by comparing the parameter with a reference parameter.

2. A method according to claim 1, wherein in (i), the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+, that are CD127- is measured.

3. A method according to claim 1, wherein in (i), the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+, that are CD62L- is measured.

4. A method according to claim 1, wherein in (i), the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+, that are CD62L- and CD127- is measured.

5. A method according to any one of the preceding claims, wherein the individual has been treated with an immune therapy composition before steps a-c are performed.

6. A method according to any one of the preceding claims, wherein the sample from the individual comprises substantially no tumor cells.

7. A method according to any one of the preceding claims, wherein the individual is without any clinical signs of a disease to be treated with the immune therapy composition.

8. A method for the preparation of an immune therapy composition comprising:
a. providing a population of cells comprising T-cells from a sample obtained from an individual that has been treated with a first immune therapy composition,
b. optionally isolating a population of antigen specific immune therapy-induced T-cells from the population obtained in step (a) and optionally measuring the frequency of antigen specific immune therapy-induced T-cells in the population of immune therapy-induced T-cells,
c. measuring in the population obtained in step (a) or (b) at least one of:
i. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+ , and
ii. the frequency of at least one of antigen specific IFNgamma producing T-cells, antigen specific TNFalpha producing T-cells and antigen specific IL-2 producing T-cells, preferably of antigen specific IFNgamma producing T-cells and antigen specific TNFalpha producing T-cells, more preferably of antigen specific IFNgamma producing T-cells, antigen specific TNFalpha producing T-cells and antigen specific IL-2 producing T-cells, more preferably of antigen specific polyfunctional T-cells, and
d. correlating a parameter measured in (b) and/or (c) with the efficacy of the first immune therapy composition by comparing the parameter with a reference parameter,
e. adapting the first immune therapy composition to the individuals needs to obtain a second immune therapy composition.

9. A method according to any one of the preceding claims, wherein the population of immune therapy-induced T-cells comprises CD4+ T-cells.

10. A method according to any one of the preceding claims, wherein the population of immune therapy-induced T-cells comprises CD8+ T-cells.

11. A method of treatment of an individual with an immune therapy composition comprising:
a. administering an immune therapy composition to an individual,
b. performing a method according to any one of the preceding claims, and optionally
c. adapting the immune therapy composition to the individuals needs.

12. A method according to any one of the preceding claims, wherein the immune therapy composition is a vaccine composition.

13. An immune therapy composition for treatment, prevention, reduction or delay of a disease or condition in an individual, wherein the immune therapy composition is to be administered to the individual, wherein subsequently a method according to any one of claims 1 to 12 is performed and wherein subsequently the immune therapy composition is adapted to the individuals needs to obtain a second immune therapy composition.

14. An immune therapy composition according to claim 13, wherein said composition is a vaccine composition.

15. Use of at least one parameter selected from the group consisting of:
a. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+ in a population comprising immune therapy-induced T-cells,
b. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+ that are CD127- in a population comprising immune therapy-induced T-cells,
c. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+ that are CD62L- in a population comprising immune therapy-induced T-cells,
d. the frequency of antigen specific KLRG1+ T-cells, preferably of antigen specific KLRG1+ T-cells that are CD154+ or of antigen specific KLRG1+ T-cells that are CD137+, more preferably of antigen specific KLRG1+ T-cells that are CD154+ and CD137+ that are CD127- and are CD62L- in a population comprising immune therapy-induced T-cells,
e. the frequency of at least one of antigen specific IFNgamma producing T-cells, antigen specific TNFalpha producing T-cells and antigen specific IL-2 producing T-cells, preferably of antigen specific IFNgamma producing T-cells and antigen specific TNFalpha producing T-cells, more preferably of antigen specific IFNgamma producing T-cells, antigen specific TNFalpha producing T-cells and antigen specific IL-2 producing T-cells, more preferably of antigen specific polyfunctional T-cells in a population comprising immune therapy-induced T-cells, and
f. the frequency of antigen specific immune therapy-induced T-cells in a population comprising immune therapy-induced T-cells,
for measuring *ex vivo* the efficacy of an immune therapy composition in an individual.
